# EUROPEAN PATENT APPLICATION

(11) **EP 3 605 227 A1**
(43) Date of publication of application: **05.02.2020**
(21) Application number: 18775122.7
(22) Date of filing: 30.03.2018
(51) Int. Cl.: G03F 7/004, C07H 3/02, G03F 7/039, G03F 7/20

(54) **RESIST COMPOSITION AND PATTERN FORMING METHOD USING SAME, AND COMPOUND AND RESIN**

(30) Priority: 31.03.2017 JP 2017072550
(71) Applicant: The School Corporation Kansai University, Suita-shi, Osaka 564-8680 (JP); Mitsubishi Gas Chemical Company, Inc., Tokyo 100-8324 (JP)
(72) Inventor: KUDO, Hiroto, Suita-shi Osaka 564-8680 (JP); SATO, Takashi, Hiratsuka-shi Kanagawa 254-0016 (JP); ECHIGO, Masatoshi, Tokyo 100-8324 (JP)
(74) Representative: Müller-Boré & Partner Patentanwälte PartG mbB
(86) International application number: PCT/JP2018/013579
(87) International publication number: WO 2018/181882

(57) **Abstract**

The present invention provides a resist composition comprising one or more tannin compounds selected from the group consisting of a tannin comprising at least one acid dissociation reactive group in the structure and a derivative thereof, and a resin obtained using the tannin or the derivative as a monomer, and a pattern formation method using the same, a compound and a resin.

## Description

### Technical Field

The present invention relates to a compound or a resin for use mainly as a lithography material, a resist composition comprising the compound or the resin, and a pattern formation method using the resist composition.

### Background Art

Conventional typical resist materials are polymer based resist materials capable of forming amorphous thin films. Examples include polymer based resist materials such as polymethyl methacrylate, polyhydroxy styrene with an acid dissociation reactive group, and polyalkyl methacrylate. A line pattern of about 45 to 100 nm is formed by irradiating a resist thin film made by coating a substrate with a solution of such a polymer based resist material with ultraviolet, far ultraviolet, electron beam, extreme ultraviolet (EUV), and X-ray or the like.

However, polymer based resist materials have a molecular weight as large as about 10,000 to 100,000 and also wide molecular weight distribution. Therefore, in lithography using a polymer based resist material, roughness occurs on a fine pattern surface; the pattern dimension becomes difficult to be controlled; and the yield decreases. Therefore, there is a limitation in miniaturization with lithography using a conventional polymer based resist material. In order to make a finer pattern, various low molecular weight resist materials have been proposed so far.

For example, an alkaline development type negative type radiation-sensitive composition (see, for example, Patent Literature 1 and Patent Literature 2) using a low molecular weight polynuclear polyphenolic compound as a main component has been suggested. As a candidate of a low molecular weight resist material having high heat resistance, an alkaline development type negative type radiation-sensitive composition (see, for example, Patent Literature 3 and Non Patent Literature 1) using a low molecular weight cyclic polyphenolic compound as a main component has been suggested as well. Moreover, as a base compound of a resist material, a polyphenol compound is known to be capable of imparting high heat resistance despite a low molecular weight and useful for improving the resolution and roughness of a resist pattern (see, for example, Non Patent Literature 2).

Also, a resist composition containing a tannin and a derivative thereof (for example, Patent Literature 4) has been proposed as a molecular resist material.

Moreover, lithography with electron beam or extreme ultraviolet (hereinafter, also referred to as "EUV") differ in reaction mechanism from general photolithography. Lithography with electron beam or EUV aims at forming fine patterns of tens of nm. Accordingly, there is a demand for a resist material having higher sensitivity for an exposing source with decrease in resist pattern dimension. Particularly, lithography with EUV needs to achieve higher sensitivity of a resist composition in terms of throughput.

As resist materials that solve these problems, inorganic resist materials having titanium, hafnium, or zirconium have been proposed (see, for example, Patent Literature 5 and Patent Literature 6).

### Citation List

### Patent Literature

Patent Literature 1: Japanese Patent Application Laid-Open No. 2005-326838
Patent Literature 2: Japanese Patent Application Laid-Open No. 2008-145539
Patent Literature 3: Japanese Patent Application Laid-Open No. 2009-173623
Patent Literature 4: Japanese Patent No. 4588551
Patent Literature 5: Japanese Patent Application Laid-Open No. 2015-75500
Patent Literature 6: Japanese Patent Application Laid-Open No. 2015-108781

### Non Patent Literature

Non Patent Literature 1: T. Nakayama, M. Nomura, K. Haga, M. Ueda: Bull. Chem. Soc. Jpn., 71, 2979 (1998)
Non Patent Literature 2: Shinji Okazaki et al., "New Trends of Photoresists", CMC Publishing Co., Ltd., September 2009, pp. 211-259

### Summary of Invention

### Technical Problem

However, the heat resistances of the compositions described in Patent Literatures 1 and 2 are not sufficient, and the shapes of the obtained resist patterns are likely to be poor. The solubilities of the compositions described in Patent Literature 3 and Non Patent Literature 1 in safe solvents used in a semiconductor production process are not sufficient. Also, the sensitivities of the compositions described in Patent Literature 3 and Non Patent Literature 1 are not sufficient, the shapes of the obtained resist patterns in some cases are poor, and thus a further improvement of low molecular weight resist materials is desired.

Non Patent Literature 2 is silent on solubility, the heat resistances of the compounds are still not sufficient, and a further improvement of heat resistance is required.

The product stability of the composition described in Patent Literature 4 is likely to be poor in such a way that, for example, properties vary among lots, because the tannin and the derivative thereof used are a mixture.

Although the resist materials described in Patent Literatures 5 and 6 have relatively high sensitivity, their sensitivities are still not sufficient. Moreover, the resist materials have disadvantages such as low solubility in safe solvents, poor storage stability, and many defects in films.

In light of these situations, an object of the present invention is to provide a resist composition which is capable of reducing film defects (thin film formability), has good storage stability and high sensitivity, and can impart a good shape to a resist pattern, a resist pattern formation method using the same, and a compound or a resin having high solubility in a safe solvent.

### Solution to Problem

The inventors have, as a result of devoted examinations to solve the above problems, found out that a resist composition comprising a compound having a specific structure can solve the above problems, and reached the present invention.

More specifically, the present invention is as follows.
[1] A resist composition comprising one or more tannin compounds selected from the group consisting of a tannin comprising at least one acid dissociation reactive group in the structure and a derivative thereof, and a resin obtained using the tannin or the derivative as a monomer.
[2] The resist composition according to the above [1], wherein the tannin compounds comprise one or more selected from the group consisting of a compound represented by the following formula (0), and a resin obtained using the compound represented by the following formula (0) as a monomer: 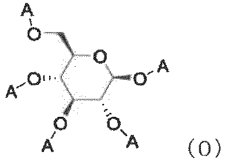 (In the formula (0), each A is independently a hydrogen atom, or any structure represented by the following formula (A), provided that at least one A is any structure represented by the following formula (A).) 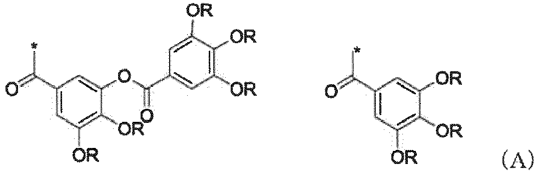 (In the formula (A), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group; and * indicates a point of attachment to the formula (0).)
[3] The resist composition according to the above [1], wherein the tannin compounds comprise one or more selected from the group consisting of a compound represented by the following formula (0-1) or the following formula (0-2), and a condensate having a structure derived from the compound represented by the following formula (0-1) and/or the following formula (0-2), and a resin obtained using the compound or the condensate as a monomer: 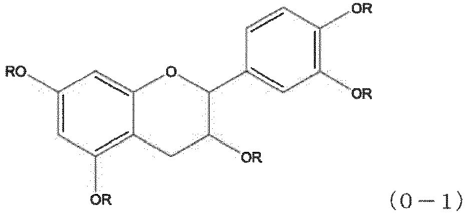 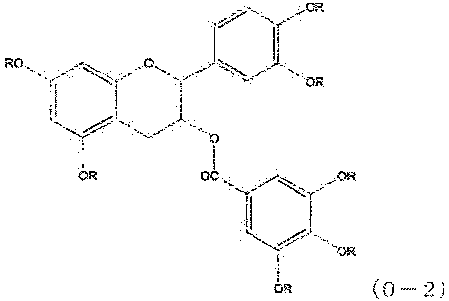 (In the formula (0-1) and the formula (0-2), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group.)
[4] The resist composition according to the above [2], wherein the compound represented by the above formula (0) is a compound represented by the following formula (1): 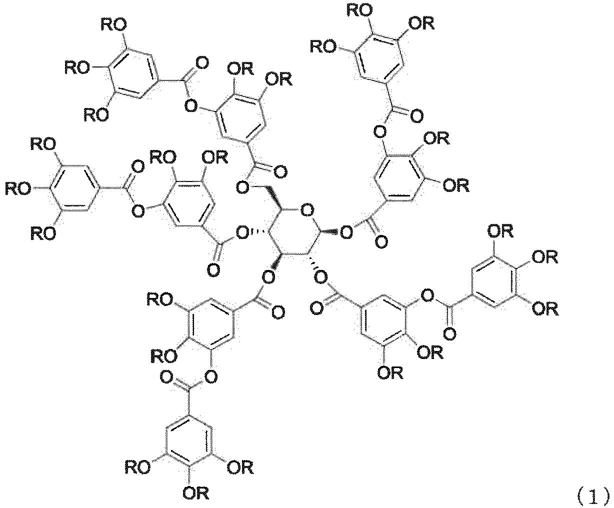 (In the formula (1), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provide that at least one R is an acid dissociation reactive group.)
[5] The resist composition according to any of the above [1] to [4], further comprising a solvent.
[6] The resist composition according to any of the above [1] to [5], further comprising an acid generating agent.
[7] The resist composition according to any of the above [1] to [6], further comprising an acid diffusion controlling agent.
[8] A method for forming a pattern, comprising the steps of: forming a resist film on a substrate using the resist composition according to any of the above [1] to [7]; exposing the resist film; and developing the resist film, thereby forming a pattern.
[9] A compound represented by the following formula (0): 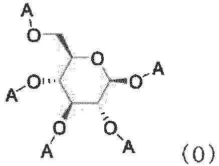 (In the formula (0), each A is independently a hydrogen atom, or any structure represented by the following formula (A), provided that at least one A is any structure represented by the following formula (A).) 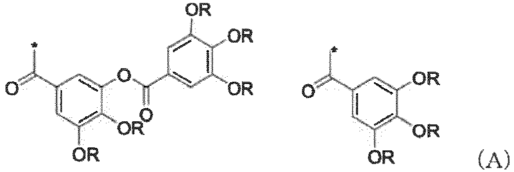 (In the formula (A), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group; and * indicates a point of attachment to the formula (0).)
[10] A resin obtained using the compound according to the above [9] as a monomer.
[11] A compound selected from the group consisting of a compound represented by the following formula (0-1) or the following formula (0-2), and a condensate having a structure derived from the compound represented by the following general formula (0-1) and/or the following general formula (0-2): 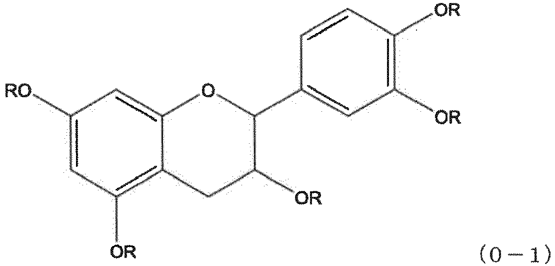 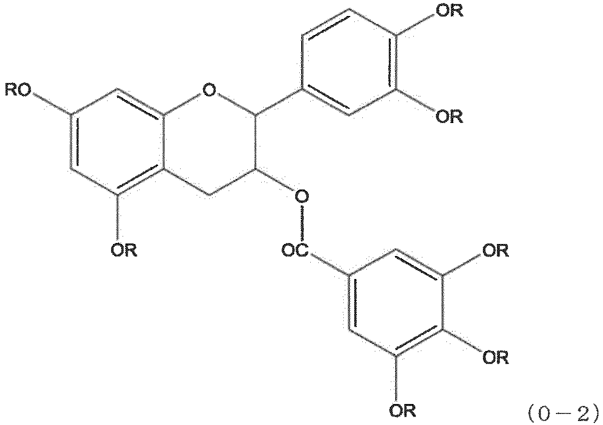 (In the formula (0-1) and the formula (0-2), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group.)
[12] A resin obtained using the compound according to the above [11] as a monomer.
[13] A compound represented by the following formula (1): 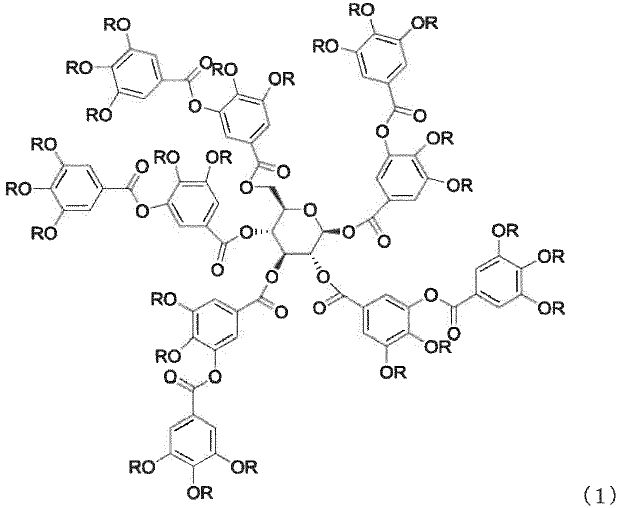 (In the formula (1), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group.)
[14] A resin obtained using the compound according to the above [13] as a monomer.

### Advantageous Effects of Invention

The present invention can provide a resist composition which is capable of reducing film defects (thin film formability), has good storage stability and high sensitivity, and can impart a good shape to a resist pattern, and a resist pattern formation method using the same.

Also, the present invention can provide a compound or a resin having high solubility in a safe solvent.

### Description of Embodiments

Hereinafter, an embodiment of the present invention will be described (hereinafter, referred to as "present embodiment"). The present embodiment is given in order to illustrate the present invention. The present invention is not limited to only the present embodiment.

### [Resist Composition]

The resist composition of the present embodiment contains one or more tannin compounds selected from the group consisting of a tannin comprising at least one acid dissociation reactive group in the structure and a derivative thereof, and a resin obtained using the tannin or the derivative as a monomer (hereinafter, they are collectively referred to as the "tannin compound according to the present embodiment"). As the tannin compound according to the present embodiment, a compound selected from a tannic acid derivative and a general tannin derivative including condensed tannin mentioned later can be used as long as the compound comprises at least one acid dissociation reactive group in the structure.

From the viewpoint of product stability, it is preferable that the resist composition of the present embodiment contains, as the tannin compound according to the present embodiment, one or more selected from the group consisting of a compound represented by the following formula (0), and a resin obtained using the compound represented by the following formula (0) as a monomer: 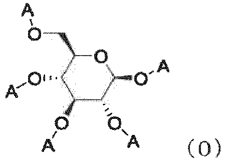 (In the formula (0), each A is independently a hydrogen atom, or any structure represented by the following formula (A), provided that at least one A is any structure represented by the following formula (A).) 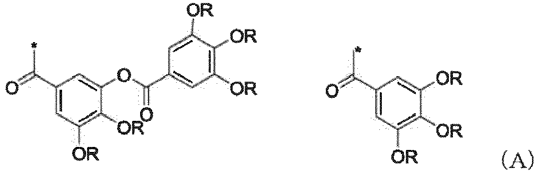 (In the formula (A), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group; and * indicates a point of attachment to the formula (0).)

As the tannin compound according to the present embodiment, one or more selected from the group consisting of a compound represented by the following formula (0-1) or the following formula (0-2), and a condensate having a structure derived from the compound represented by the following formula (0-1) and/or the following formula (0-2), and a resin obtained using the condensate as a monomer (hereinafter, they are also collectively referred to as a "condensed tannin compound") can be used from the viewpoint of easy availability. A specific form of the condensed tannin compound will be mentioned later. 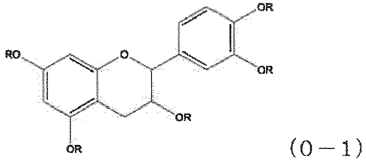 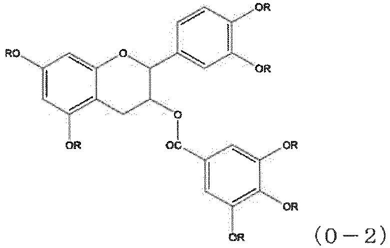 (In the formula (0-1) and the formula (0-2), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group.)

Likewise, from the viewpoint of easy availability, it is also preferable to use, as the tannin compound according to the present embodiment, one or more selected from the group consisting of a compound represented by the following formula (0-3), and a resin obtained using the compound as a monomer: 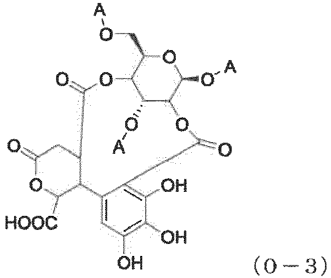 (In the formula (0-3), each A is independently a hydrogen atom, or any structure represented by the following formula (A), provided that at least one A is any structure represented by the following formula (A).) 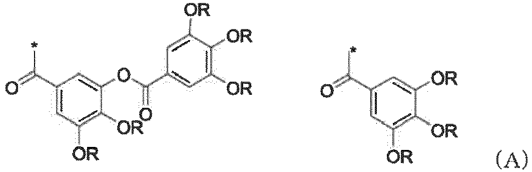 (In the formula (A), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group; and * indicates a point of attachment to the formula (0-3).)

Moreover, as the tannin compound according to the present embodiment, a general tannin derivative such as a compound represented by the following formula (0-4) can also be used: 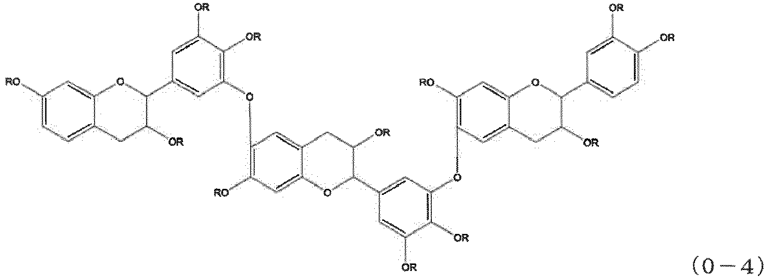 (In the formula (0-4), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group.)

### [Compound Represented by Formula (0)]

The compound represented by the formula (0) according to the present embodiment is as follows: 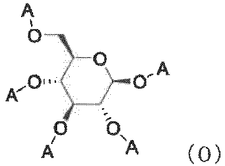 In the above formula (0), each A is independently a hydrogen atom, or any structure represented by the following formula (A), provided that at least one A is any structure represented by the following formula (A). 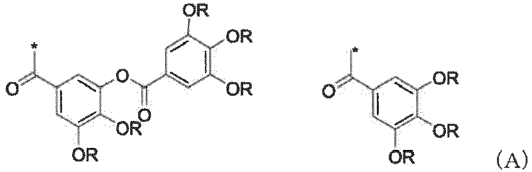 In the above formula (A), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group; and * indicates a point of attachment to the formula (0).

The resist composition of the present embodiment can contain, as the tannin compound according to the present embodiment, one or more selected from the group consisting of a compound having a specific structure and a resin obtained using the compound as a monomer. The compound represented by the formula (0) is a tannic acid derivative. Since tannic acid contains many hydroxy groups in the structure, the solubility of the tannic acid derivative, which is a derivative thereof, is easy to control, and the higher sensitivity thereof can be expected in lithography.

Examples of the compound represented by the above formula (0) include, but not particularly limited to, a compound represented by the following formula (0') or formula (1): 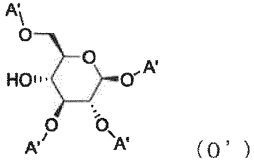 In the above formula (0'), each A' is independently any structure represented by the following formula (A): 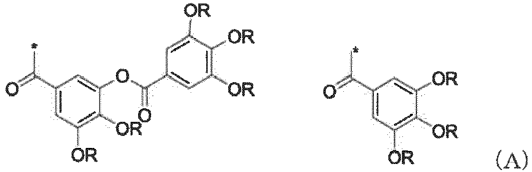 In the above formula (A), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group; and * indicates a point of attachment to the formula (0'). 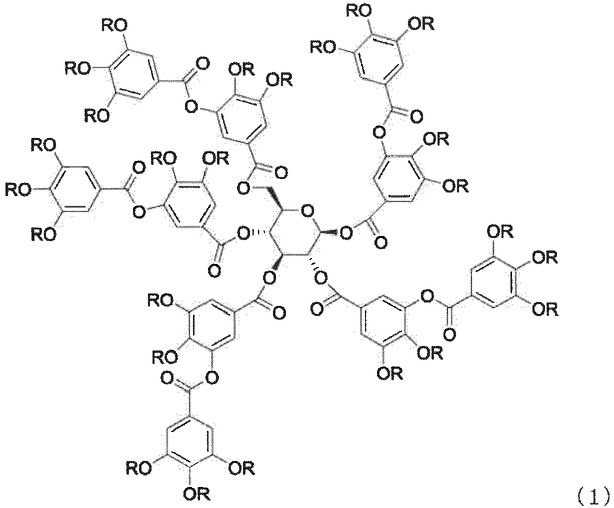 In the above formula (1), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group.

### [Condensed Tannin Compound]

As mentioned above, as the tannin compound according to the present embodiment, a condensed tannin compound can be used. Examples of the condensed tannin compound according to the present embodiment include at least one selected from compounds described below. The condensate described below in (3) may have a structure derived from only any one of compounds represented by the formula (0-1) and the formula (0-2), or may have structures derived from both the formulae. Furthermore, the number of structures derived from each formula contained in the above condensate is not particularly limited.
(1) A compound represented by the following formula (0-1)
(2) A compound represented by the following formula (0-2)
(3) A condensate having a structure derived from the compound represented by the following formula (0-1) and/or the following formula (0-2)
(4) A resin obtained using any of the above (1) to (3) as a monomer 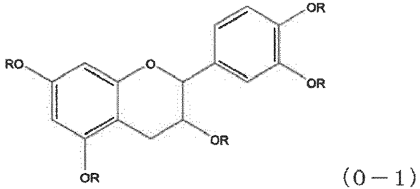 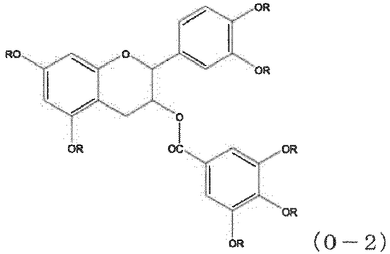
(In the formula (0-1) and the formula (0-2), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group.)

Examples of the compound that can be used as the condensed tannin compound according to the present embodiment include, but not particularly limited to, respective compounds represented by the following formulae (2-1) to (2-5): 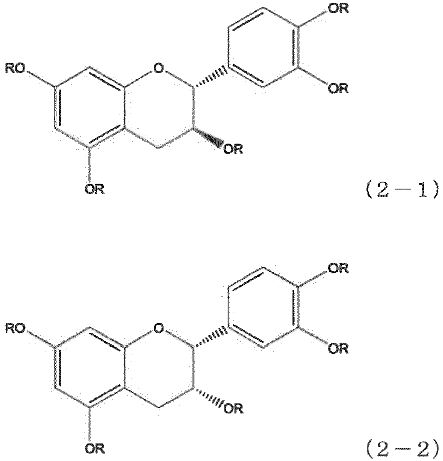 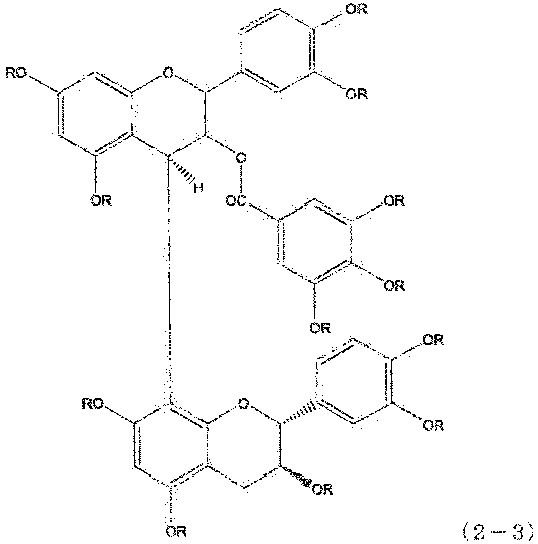 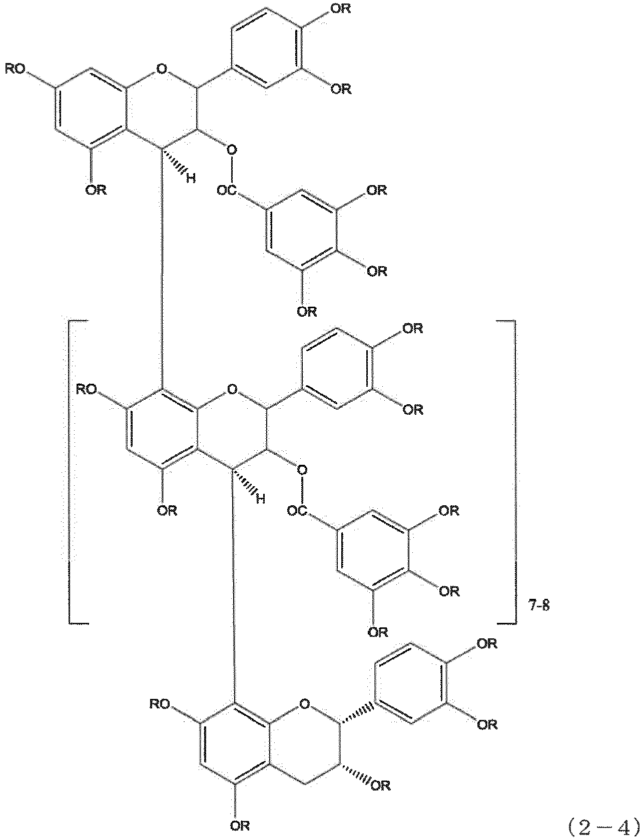 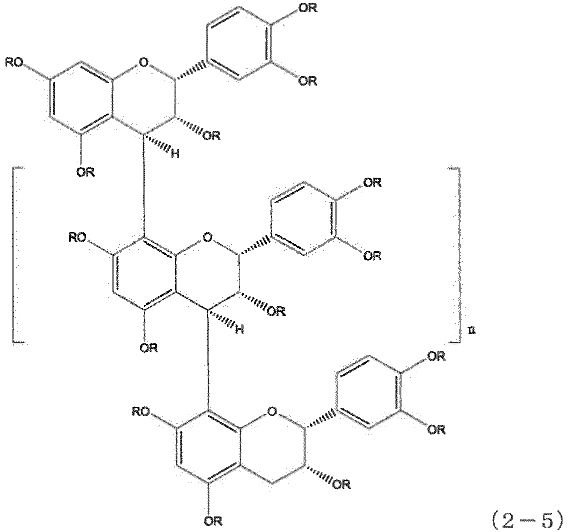 In the above formulae (2-1) to (2-5), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group. However, at least one R is an acid dissociation reactive group.

### [Other Compounds That Can Be Suitably Used]

As the tannin compound according to the present embodiment, a condensed tannin derivative such as a quebracho tannin derivative represented by the following formula (0-3) or a wattle tannin derivative represented by the following formula (0-4) can also be suitably used: 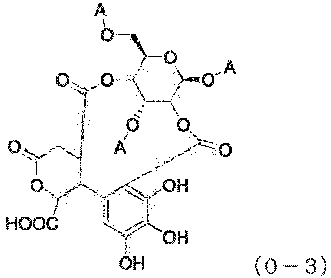 (In the formula (0-3), each A is independently a hydrogen atom, or any structure represented by the following formula (A), provided that at least one A is any structure represented by the following formula (A).) 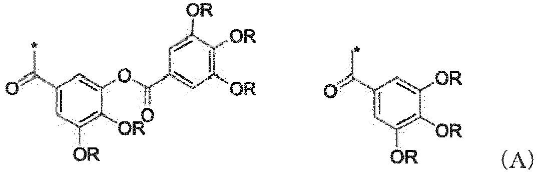 (In the formula (A), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group; and * indicates a point of attachment to the formula (0-3).) 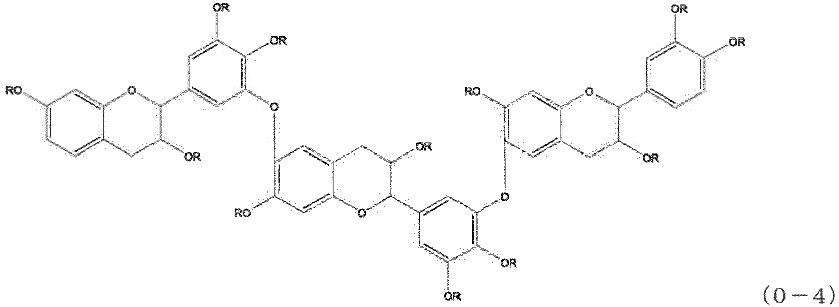 In the formula (0-4), each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group.

The chemical structure of the compound contained in the resist composition of the present embodiment can be determined by ¹H-NMR analysis.

Since the tannin compound contained in the resist composition of the present embodiment contains at least one acid dissociation reactive group, as shown in the above formula (0) and the like, high sensitivity can be expected in lithography. Also, the tannin compound has a benzene skeleton and is therefore excellent in heat resistance. Furthermore, since tannic acid derived from a natural product can be used as a raw material, the tannin compound can be inexpensively obtained.

Each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, and at least one R is an acid dissociation reactive group.

In the present specification, the term "substituted" means that one or more hydrogen atoms in a functional group are substituted with a halogen atom, a hydroxy group, a cyano group, a nitro group, a heterocyclic group, a linear aliphatic hydrocarbon group having 1 to 20 carbon atoms, a branched aliphatic hydrocarbon group having 3 to 20 carbon atoms, a cyclic aliphatic hydrocarbon group having 3 to 20 carbon atoms, an aryl group having 6 to 20 carbon atoms, an aralkyl group having 7 to 30 carbon atoms, an alkoxy group having 1 to 20 carbon atoms, an amino group having 0 to 20 carbon atoms, an alkenyl group having 2 to 20 carbon atoms, an acyl group having 1 to 20 carbon atoms, an alkoxycarbonyl group having 2 to 20 carbon atoms, an alkyloyloxy group having 1 to 20 carbon atoms, an aryloyloxy group having 7 to 30 carbon atoms, an alkylsilyl group having 1 to 20 carbon atoms, or the like unless otherwise defined.

Examples of the unsubstituted linear aliphatic hydrocarbon group having 1 to 20 carbon atoms include a methyl group, an ethyl group, a propyl group, a butyl group, a pentyl group, a hexyl group, an octyl group, a decyl group, a dodecyl group, a hexadecyl group, and an octadecyl group.

Examples of the substituted linear aliphatic hydrocarbon group having 1 to 20 carbon atoms include a fluoromethyl group, a 2-hydroxyethyl group, a 3-cyanopropyl group, and a 20-nitrooctadecyl group.

Examples of the unsubstituted branched aliphatic hydrocarbon group having 3 to 20 carbon atoms include an isopropyl group, an isobutyl group, a tertiary butyl group, a neopentyl group, a 2-hexyl group, a 2-octyl group, a 2-decyl group, a 2-dodecyl group, a 2-hexadecyl group, and a 2-octadecyl group.

Examples of the substituted branched aliphatic hydrocarbon group having 3 to 20 carbon atoms include a 1-fluoroisopropyl group and a 1-hydroxy-2-octadecyl group.

Examples of the unsubstituted cyclic aliphatic hydrocarbon group having 3 to 20 carbon atoms include a cyclopropyl group, a cyclobutyl group, a cyclopentyl group, a cyclohexyl group, a cyclooctyl group, a cyclodecyl group, a cyclododecyl group, a cyclohexadecyl group, and a cyclooctadecyl group.

Examples of the substituted cyclic aliphatic hydrocarbon group having 3 to 20 carbon atoms include a 2-fluorocyclopropyl group and a 4-cyanocyclohexyl group.

Examples of the unsubstituted aryl group having 6 to 20 carbon atoms include a phenyl group and a naphthyl group.

Examples of the substituted aryl group having 6 to 20 carbon atoms include a 4-isopropylphenyl group, a 4-cyclohexylphenyl group, a 4-methylphenyl group, and a 6-fluoronaphthyl group.

Examples of the unsubstituted alkenyl group having 2 to 20 carbon atoms include a vinyl group, a propynyl group, a butynyl group, a pentynyl group, a hexynyl group, an octynyl group, a decynyl group, a dodecynyl group, a hexadecynyl group, and an octadecynyl group.

Examples of the substituted alkenyl group having 2 to 20 carbon atoms include a chloropropynyl group.

Examples of the halogen atom include a fluorine atom, a chlorine atom, a bromine atom, and an iodine atom.

### - Acid Dissociation Reactive Group -

In the present specification, the "acid dissociation reactive group" refers to a characteristic group that is cleaved in the presence of an acid to cause a change such as an alkali soluble group. Examples of the alkali soluble group include, but not particularly limited to, a phenolic hydroxy group, a carboxyl group, a sulfonic acid group, and a hexafluoroisopropanol group. Among them, a phenolic hydroxy group and a carboxyl group are preferable, and a phenolic hydroxy group is particularly preferable, from the viewpoint of the easy availability of an introduction reagent.

The acid dissociation reactive group preferably has the property of causing chained cleavage reaction in the presence of an acid, for achieving pattern formation with high sensitivity and high resolution. The acid dissociation reactive group is not particularly limited, but can be arbitrarily selected for use from among, for example, those proposed in hydroxystyrene based resins, (meth)acrylic acid based resins, and the like for use in chemically amplified resist compositions for KrF or ArF.

Preferable examples of the acid dissociation reactive group include a group selected from the group consisting of a substituted methyl group, a 1-substituted ethyl group, a 1-substituted n-propyl group, a 1-branched alkyl group, a silyl group, an acyl group, a 1-substituted alkoxymethyl group, a cyclic ether group, an alkoxycarbonyl group, and an alkoxycarbonylalkyl group which have the property of being dissociated by an acid. From the viewpoint of roughness, it is preferable that the acid dissociation reactive group has no crosslinkable functional group.

The substituted methyl group is not particularly limited, but is usually a substituted methyl group having 2 to 20 carbon atoms and is preferably a substituted methyl group having 4 to 18 carbon atoms and more preferably a substituted methyl group having 6 to 16 carbon atoms. Specific examples of the substituted methyl group can include, but not limited to, a methoxymethyl group, a methylthiomethyl group, an ethoxymethyl group, a n-propoxymethyl group, an isopropoxymethyl group, a n-butoxymethyl group, a t-butoxymethyl group, a 2-methylpropoxymethyl group, an ethylthiomethyl group, a methoxyethoxymethyl group, a phenyloxymethyl group, a 1-cyclopentyloxymethyl group, a 1-cyclohexyloxymethyl group, a benzylthiomethyl group, a phenacyl group, a 4-bromophenacyl group, a 4-methoxyphenacyl group, a piperonyl group, and a substituent group represented by the following formula (13-1). 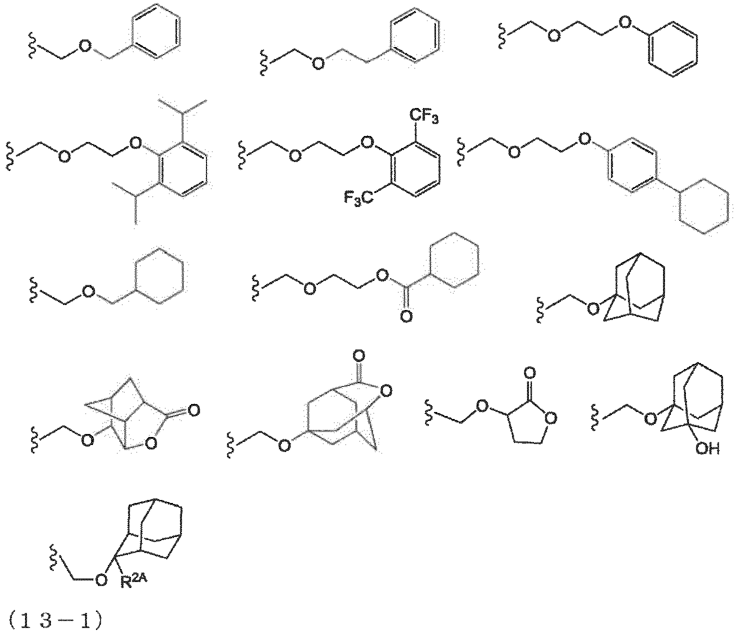 In the above formula (13-1), R^{2A} is an alkyl group having 1 to 4 carbon atoms. Specific examples of R^{2A} include, but not limited to, a methyl group, an ethyl group, an isopropyl group, a n-propyl group, a t-butyl group, and a n-butyl group.

The 1-substituted ethyl group is not particularly limited, but is usually a 1-substituted ethyl group having 3 to 20 carbon atoms and is preferably a 1-substituted ethyl group having 5 to 18 carbon atoms and more preferably a substituted ethyl group having 7 to 16 carbon atoms. Specific examples of the 1-substituted ethyl group can include, but not limited to, a 1-methoxyethyl group, 1-methylthioethyl group, a 1,1-dimethoxyethyl group, a 1-ethoxyethyl group, a 1-ethylthioethyl group, a 1,1-diethoxyethyl group, a n-propoxyethyl group, an isopropoxyethyl group, a n-butoxyethyl group, a t-butoxyethyl group, a 2-methylpropoxyethyl group, a 1-phenoxyethyl group, a 1-phenylthioethyl group, a 1,1-diphenoxyethyl group, a 1-cyclopentyloxyethyl group, a 1-cyclohexyloxyethyl group, a 1-phenylethyl group, a 1,1-diphenylethyl group, and a substituent group represented by the following formula (13-2) : 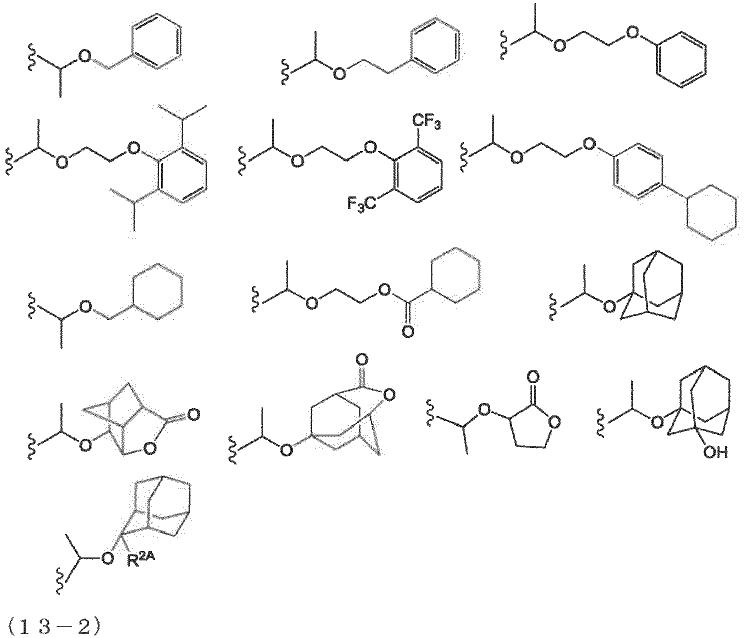 In the above formula (13-2), R^{2A} is as defined above.

The 1-substituted n-propyl group is not particularly limited, but is usually a 1-substituted n-propyl group having 4 to 20 carbon atoms and is preferably a 1-substituted n-propyl group having 6 to 18 carbon atoms and more preferably a 1-substituted n-propyl group having 8 to 16 carbon atoms. Specific examples of the 1-substituted n-propyl group can include, but not limited to, a 1-methoxy-n-propyl group and a 1-ethoxy-n-propyl group.

The 1-branched alkyl group is not particularly limited, but is usually a 1-branched alkyl group having 3 to 20 carbon atoms and is preferably a 1-branched alkyl group having 5 to 18 carbon atoms and more preferably a branched alkyl group having 7 to 16 carbon atoms. Specific examples of the 1-branched alkyl group can include, but not limited to, an isopropyl group, a sec-butyl group, a tert-butyl group, a 1,1-dimethylpropyl group, a 1-methylbutyl group, a 1,1-dimethylbutyl group, a 2-methyladamantyl group, and a 2-ethyladamantyl group.

The silyl group is not particularly limited, but is usually a silyl group having 1 to 20 carbon atoms and is preferably a silyl group having 3 to 18 carbon atoms and more preferably a silyl group having 5 to 16 carbon atoms. Specific examples of the silyl group can include, but not limited to, a trimethylsilyl group, an ethyldimethylsilyl group, a methyldiethylsilyl group, a triethylsilyl group, a tert-butyldimethylsilyl group, a tert-butyldiethylsilyl group, a tert-butyldiphenylsilyl group, a tri-tert-butylsilyl group, and a triphenylsilyl group.

The acyl group is not particularly limited, but is usually an acyl group having 2 to 20 carbon atoms and is preferably an acyl group having 4 to 18 carbon atoms and more preferably an acyl group having 6 to 16 carbon atoms. Specific examples of the acyl group can include, but not limited to, an acetyl group, a phenoxyacetyl group, a propionyl group, a butyryl group, a heptanoyl group, a hexanoyl group, a valeryl group, a pivaloyl group, an isovaleryl group, a lauroyl group, an adamantylcarbonyl group, a benzoyl group, and a naphthoyl group.

The 1-substituted alkoxymethyl group is not particularly limited, but is usually a 1-substituted alkoxymethyl group having 2 to 20 carbon atoms and is preferably a 1-substituted alkoxymethyl group having 4 to 18 carbon atoms and more preferably a 1-substituted alkoxymethyl group having 6 to 16 carbon atoms. Specific examples of the 1-substituted alkoxymethyl group can include, but not limited to, a 1-cyclopentylmethoxymethyl group, a 1-cyclopentylethoxymethyl group, a 1-cyclohexylmethoxymethyl group, a 1-cyclohexylethoxymethyl group, a 1-cyclooctylmethoxymethyl group, and a 1-adamantylmethoxymethyl group.

The cyclic ether group is not particularly limited, but is usually a cyclic ether group having 2 to 20 carbon atoms and is preferably a cyclic ether group having 4 to 18 carbon atoms and more preferably a cyclic ether group having 6 to 16 carbon atoms. Specific examples of the cyclic ether group can include, but not limited to, a tetrahydropyranyl group, a tetrahydrofuranyl group, a tetrahydrothiopyranyl group, a tetrahydrothiofuranyl group, a 4-methoxytetrahydropyranyl group, and a 4-methoxytetrahydrothiopyranyl group.

The alkoxycarbonyl group is usually an alkoxycarbonyl group having 2 to 20 carbon atoms and is preferably an alkoxycarbonyl group having 4 to 18 carbon atoms and more preferably an alkoxycarbonyl group having 6 to 16 carbon atoms. Specific examples of the alkoxycarbonyl group can include, but not limited to, a methoxycarbonyl group, an ethoxycarbonyl group, a n-propoxycarbonyl group, an isopropoxycarbonyl group, a n-butoxycarbonyl group, a tert-butoxycarbonyl group, and a group of acid dissociation reactive groups represented by the following formula (13-3) wherein n = 0.

The alkoxycarbonylalkyl group is not particularly limited, but is usually an alkoxycarbonylalkyl group having 2 to 20 carbon atoms and is preferably an alkoxycarbonylalkyl group having 4 to 18 carbon atoms and more preferably an alkoxycarbonylalkyl group having 6 to 16 carbon atoms. Specific examples of the alkoxycarbonylalkyl group can include, but not limited to, a methoxycarbonylmethyl group, an ethoxycarbonylmethyl group, a n-propoxycarbonylmethyl group, an isopropoxycarbonylmethyl group, a n-butoxycarbonylmethyl group, and a group of acid dissociation reactive groups represented by the following formula (13-3) wherein n = 1 to 4: 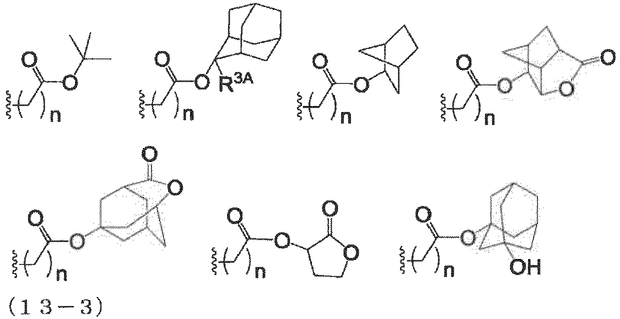 In the above formula (13-3), R^{3A} is a hydrogen atom or a linear or branched alkyl group having 1 to 4 carbon atoms; and n is an integer of 0 to 4.

Among these acid dissociation reactive groups, a substituted methyl group, a 1-substituted ethyl group, a 1-substituted alkoxymethyl group, a cyclic ether group, an alkoxycarbonyl group, and an alkoxycarbonylalkyl group are preferable. From the viewpoint of exerting higher sensitivity, a substituted methyl group, a 1-substituted ethyl group, an alkoxycarbonyl group, and an alkoxycarbonylalkyl group are more preferable, and an acid dissociation reactive group having a structure selected from a cycloalkane having 3 to 12 carbon atoms, a lactone, and an aromatic ring having 6 to 12 carbon atoms is further preferable.

The cycloalkane having 3 to 12 carbon atoms may be monocyclic or polycyclic and is preferably polycyclic.

Specific examples of the cycloalkane having 3 to 12 carbon atoms include, but not limited to, monocycloalkanes, bicycloalkanes, tricycloalkanes, and tetracycloalkanes. More specific examples thereof include, but not limited to: monocycloalkanes such as cyclopropane, cyclobutane, cyclopentane, and cyclohexane; and polycycloalkanes such as adamantane, norbornane, isobornane, tricyclodecane, and tetracyclodecane. Among them, adamantane, tricyclodecane, and tetracyclodecane are preferable, and adamantane and tricyclodecane are more preferable. The cycloalkane having 3 to 12 carbon atoms may have a substituent group.

Examples of the lactone include, but not limited to, cycloalkane groups having 3 to 12 carbon atoms having a butyrolactone or lactone group.

Examples of the aromatic ring having 6 to 12 carbon atoms include, but not limited to, a benzene ring, a naphthalene ring, an anthracene ring, a phenanthrene ring, and a pyrene ring. A benzene ring and a naphthalene ring are preferable, and a naphthalene ring is more preferable.

Among those described above, an acid dissociation reactive group selected from the group consisting of groups represented by the following formula (13-4) is preferable because of a tendency that resolution is higher: 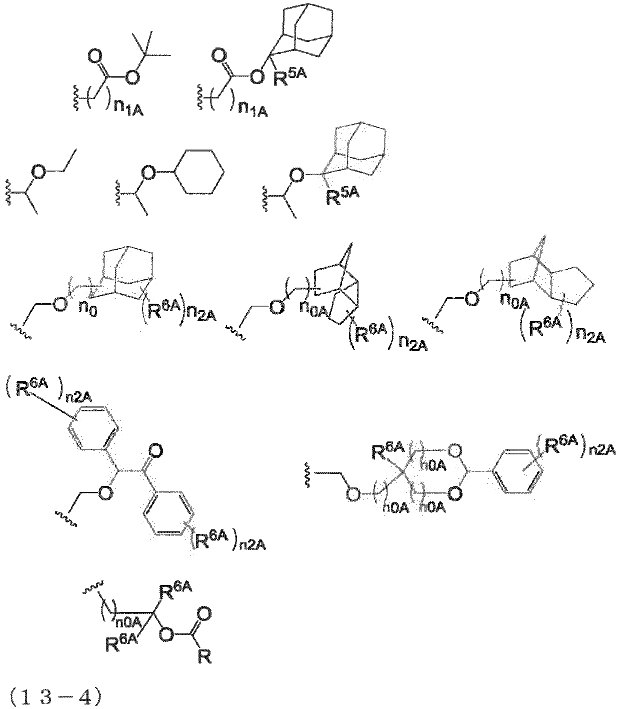 In the above formula (13-4), R^{5A} is a hydrogen atom or a linear or branched alkyl group having 1 to 4 carbon atoms; R^{6A} is a hydrogen atom, a linear or branched alkyl group having 1 to 4 carbon atoms, a cyano group, a nitro group, a heterocyclic group, a halogen atom, or a carboxyl group; n_{1A} is an integer of 0 to 4; n_{2A} is an integer of 1 to 5; and n_{0A} is an integer of 0 to 4.

Specific examples of the acid dissociation reactive group represented by R in the compounds represented by the above formula (A), formula (A'), and formulae (0-1) to (0-4), etc. include the following structural formulae. R in the following formula (12) is the same as R in the formula (A), etc. a, b, c, and d in the following formulae (15) to (17) and formulae (19) to (22) indicate the molar ratio of any constitutional unit. In the formulae, * indicates a point of attachment to a main structure in each formula.

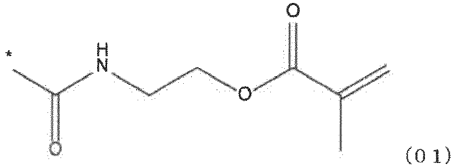

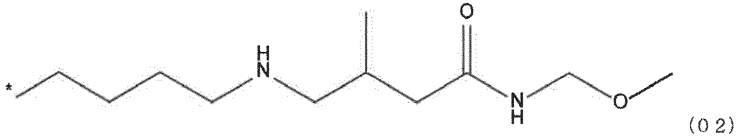

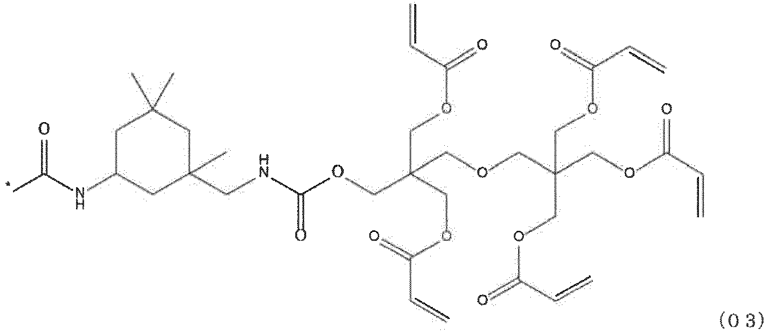

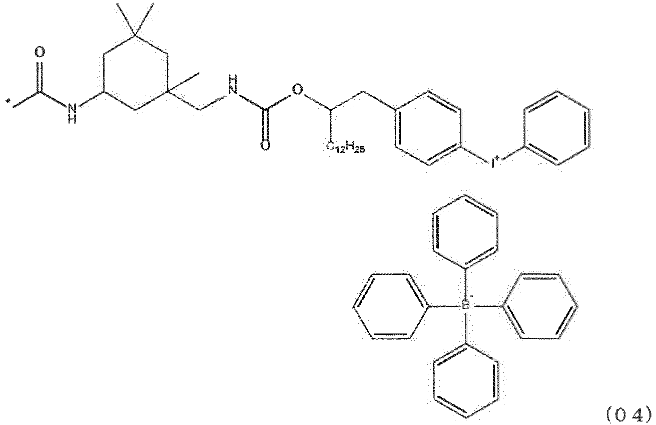

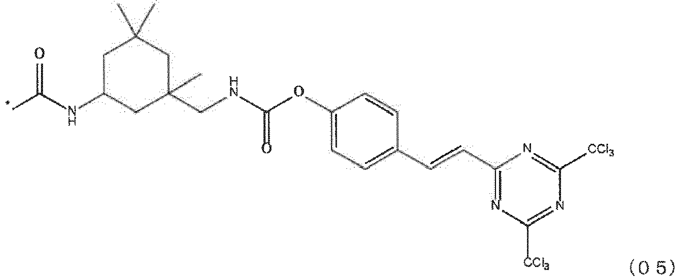

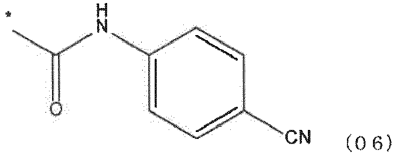

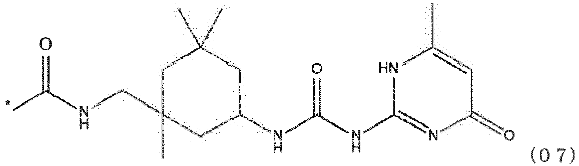

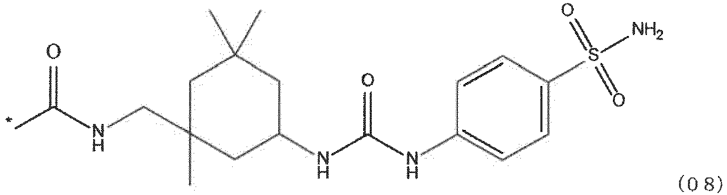

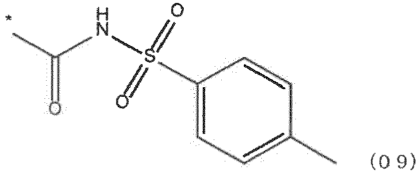

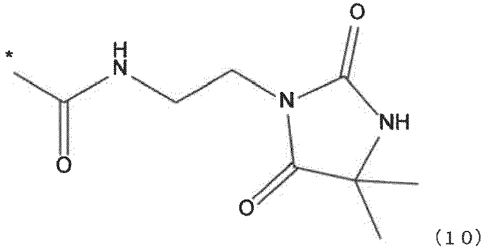

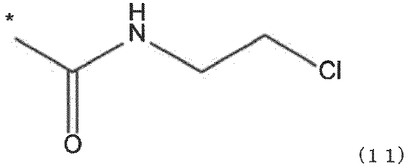

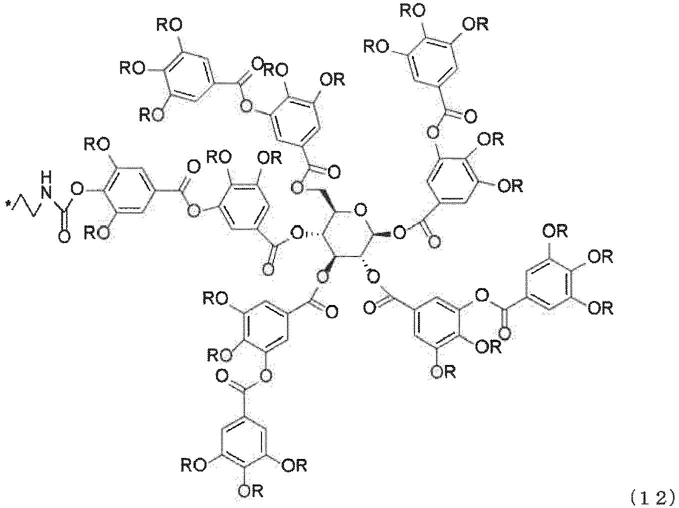

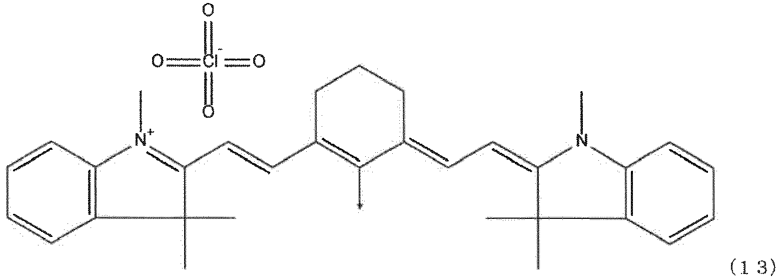

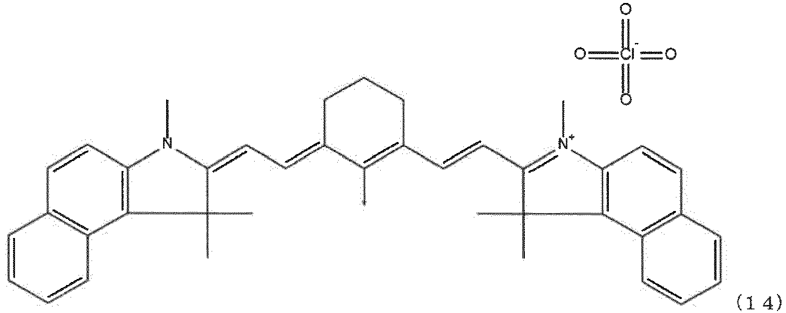

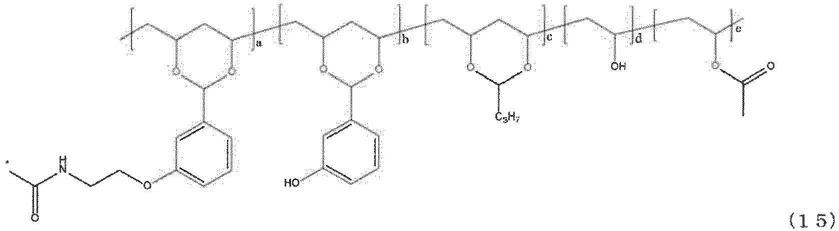

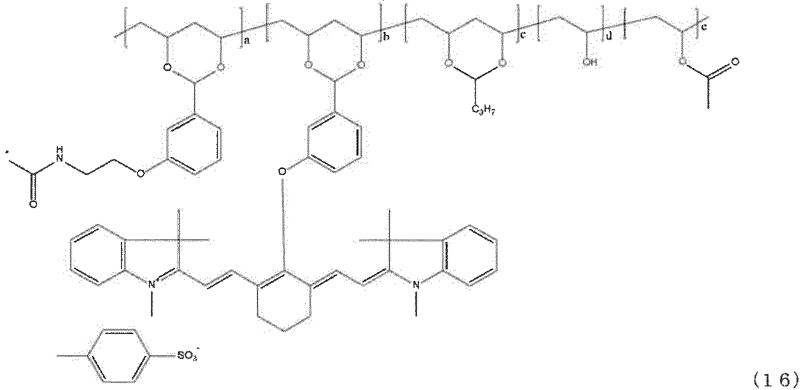

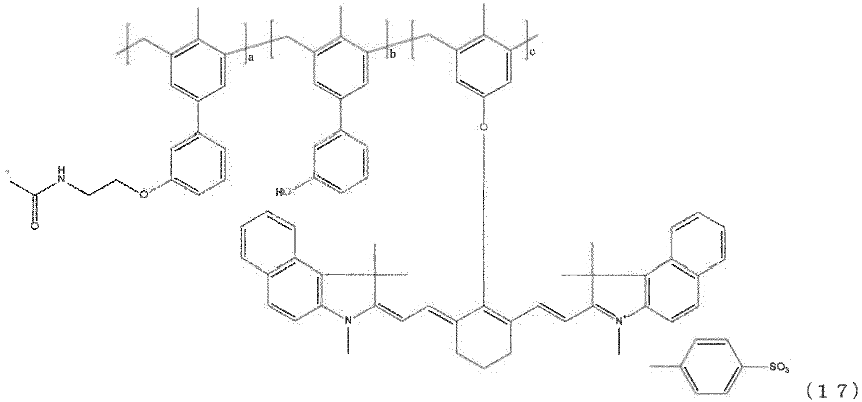

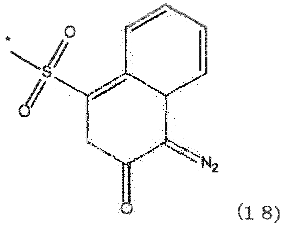

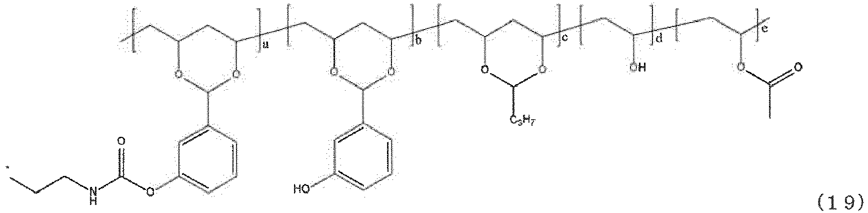

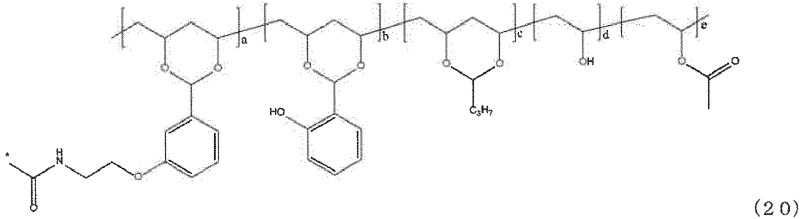

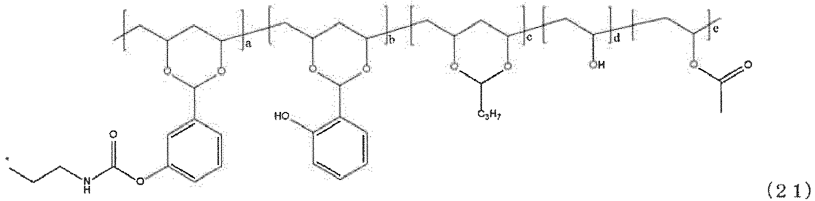

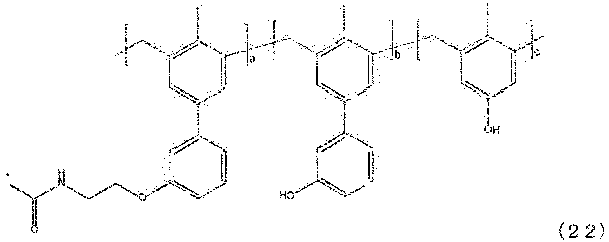

### [Method for Producing Compound Represented by Formula (0) or (1)]

A method for producing the compound represented by the formula (0) or (1) is not particularly limited, and the compound is obtained, for example, by introducing an acid dissociation reactive group to at least one phenolic hydroxy group of tannic acid represented by the following formula (0A) or (1A) : 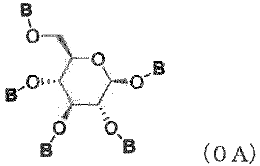 In the formula (0A), each B is independently a hydrogen atom, or any structure represented by the following formula (B). However, at least one B is any structure represented by the following formula (B). In the following formula (B), * indicates a point of attachment to the formula (0A).

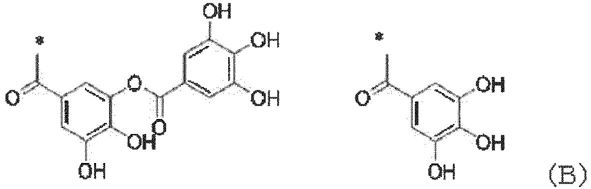

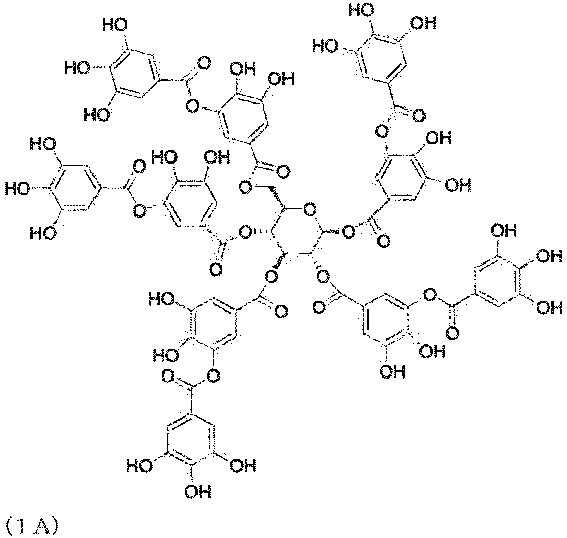

A method for producing the compound represented by the formula (0-1) or the following formula (0-2), or the condensate having a structure derived from the compound represented by the following formula (0-1) and/or the following formula (0-2) is not particularly limited, and the compound or the condensate is obtained, for example, by introducing an acid dissociation reactive group to at least one phenolic hydroxy group of condensed tannin selected from a compound represented by the following formula (0-1A) or the following formula (0-2A), and a condensate having a structure derived from the compound represented by the following formula (0-1A) and/or the following formula (0-2A): 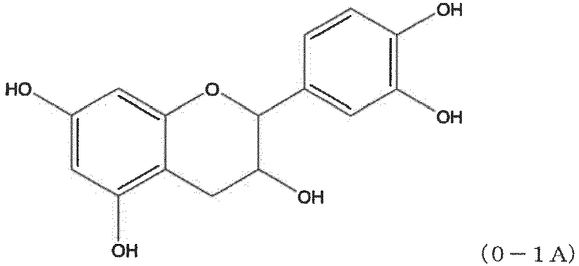 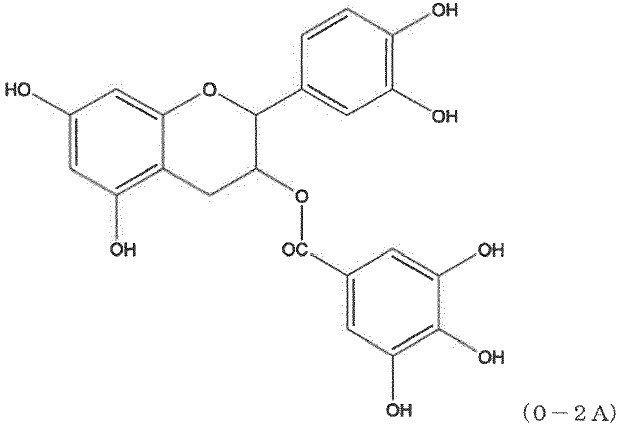 In the present embodiment, a publicly known method can be applied to a method for introducing an acid dissociation reactive group to a phenolic hydroxy group.

A compound for introducing the acid dissociation reactive group can be synthesized by a publicly known method or easily obtained. Examples thereof include, but not particularly limited to, acid chlorides, acid anhydrides, active carboxylic acid derivative compounds such as dicarbonate, alkyl halides, vinyl alkyl ethers, dihydropyran, and halocarboxylic acid alkyl esters.

An acid dissociation reactive group can be introduced to at least one phenolic hydroxy group of the tannic acid (1A), for example, as described below.

The tannic acid (1A) is dissolved or suspended in an aprotic solvent such as acetone, tetrahydrofuran (THF), propylene glycol monomethyl ether acetate, dimethylacetamide, or N-methylpyrrolidone. Subsequently, a vinyl alkyl ether such as ethyl vinyl ether, or dihydropyran is added to the solution or the suspension, and the mixture is reacted at 20 to 60°C at normal pressure for 6 to 72 hours in the presence of an acid catalyst such as pyridinium-p-toluenesulfonate. The reaction solution is neutralized with an alkali compound and added to distilled water to precipitate a white solid. Then, the separated white solid can be washed with distilled water and dried to obtain the compound represented by the above formula (1).

The acid catalyst is not particularly limited, and inorganic acids and organic acids which are well-known acid catalysts can be used. Examples of the acid catalyst include, but not particularly limited to, inorganic acids such as hydrochloric acid, sulfuric acid, phosphoric acid, hydrobromic acid, and hydrofluoric acid; organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, and naphthalenedisulfonic acid; Lewis acids such as zinc chloride, aluminum chloride, iron chloride, and boron trifluoride; and solid acids such as tungstosilicic acid, tungstophosphoric acid, silicomolybdic acid, and phosphomolybdic acid. Among them, organic acids and solid acids are preferable from the viewpoint of production, and hydrochloric acid or sulfuric acid is more preferable from the viewpoint of production such as easy availability and handleability. The acid catalysts can be used alone as one kind, or can be used in combination of two or more kinds.

Also, an acid dissociation reactive group can be introduced to at least one phenolic hydroxy group of the tannic acid (1A), for example, as described below.

The tannic acid (1A) is dissolved or suspended in an aprotic solvent such as acetone, THF, propylene glycol monomethyl ether acetate, dimethylacetamide, or N-methylpyrrolidone. Subsequently, an alkyl halide such as ethyl chloromethyl ether or a halocarboxylic acid alkyl ester such as methyladamantyl bromoacetate is added to the solution or the suspension, and the mixture is reacted at 20 to 110°C at normal pressure for 6 to 72 hours in the presence of a basic catalyst such as potassium carbonate. The reaction solution is neutralized with an acid such as hydrochloric acid and added to distilled water to precipitate a white solid. Then, the separated white solid can be washed with distilled water and dried to obtain the compound represented by the above formula (1).

The basic catalyst is not particularly limited and can be arbitrarily selected from well-known basic catalysts, and examples include: inorganic bases such as metal hydrides (alkali metal hydrides such as sodium hydride and potassium hydride, etc.), metal alcohol salts (alcohol salts of alkali metals such as sodium methoxide and potassium ethoxide), metal hydroxides (alkali metal or alkaline earth metal hydroxides such as sodium hydroxide and potassium hydroxide, etc.), metal carbonates (alkali metal or alkaline earth metal carbonates such as sodium carbonate and potassium carbonate, etc.), and alkali metal or alkaline earth metal bicarbonates such as sodium bicarbonate and potassium bicarbonate; and organic bases such as amines (for example, tertiary amines (trialkylamines such as triethylamine, aromatic tertiary amines such as N,N-dimethylaniline, and heterocyclic tertiary amines such as 1-methylimidazole), and carboxylic acid metal salts (acetic acid alkali metal or alkaline earth metal salts such as sodium acetate and calcium acetate, etc.). Among them, sodium carbonate or potassium carbonate is preferable from the viewpoint of production such as easy availability and handleability. The basic catalysts can be used alone as one kind, or can be used in combination of two or more kinds.

In the present embodiment, a method for obtaining the tannic acid (0A) or (1A), or the condensed tannin (the compound represented by the formula (0-1A) or (0-2A), and the condensate having a structure derived from the compound represented by formula (0-1A) and/or (0-2A)) used as a raw material is not particularly limited, and commercially available tannic acid or condensed tannin can be used.

### [Resin Obtained Using, as Monomer, Compound Represented by Formula (0), Formula (1),Formula (0-1), or Formula (0-2), or Condensate Having Structure Derived from Compound Represented by Formula (0-1) and/or Formula (0-2)]

The resin according to the present embodiment is obtained, for example, by reacting the compound represented by the formula (0), the formula (1), the formula (0-1), or the formula (0-2), or the condensate having a structure derived from the compound represented by the formula (0-1) and/or the formula (0-2) (hereinafter, they are collectively referred to as a "compound represented by the formula (0), etc.") with a crosslinking compound reactable with the compound represented by the formula (0), etc.

As the crosslinking compound, a publicly known compound can be used without particular limitations as long as it can oligomerize or polymerize the compound represented by the formula (0), etc. Specific examples thereof include, but not particularly limited to, aldehydes, ketones, carboxylic acids, carboxylic acid halides, halogen-containing compounds, amino compounds, imino compounds, isocyanates, and unsaturated hydrocarbon group-containing compounds.

### [Resist permanent film]

The resist composition of the present embodiment can also be used to prepare a resist permanent film. The resist permanent film prepared by coating with the above composition is suitable as a permanent film that also remains in a final product, if required, after formation of a resist pattern. Specific examples of the permanent film include, but not particularly limited to, in relation to semiconductor devices, solder resists, package materials, underfill materials, package adhesive layers for circuit elements and the like, and adhesive layers between integrated circuit elements and circuit substrates, and in relation to thin displays, thin film transistor protecting films, liquid crystal color filter protecting films, black matrixes, and spacers. Particularly, the permanent film made of the above composition is excellent in heat resistance and moisture resistance and furthermore, also has the excellent advantage that contamination by sublimable components is reduced. Particularly, for a display material, a material that achieves all of high sensitivity, high heat resistance, and hygroscopic reliability with reduced deterioration in image quality due to significant contamination can be obtained.

In the case of using the above composition for the purpose of preparing resist permanent films, a curing agent as well as, if required, various additive agents such as other resins, a surfactant, a dye, a filler, a crosslinking agent, and a dissolution promoting agent can be added and dissolved in an organic solvent to prepare a composition for resist permanent films.

The above composition for resist permanent films can be prepared by adding each of the above components and mixing them using a stirrer or the like. When the above composition for resist underlayer films or composition for resist permanent films contains a filler or a pigment, it can be prepared by dispersion or mixing using a dispersion apparatus such as a dissolver, a homogenizer, and a three-roll mill.

### [Method for Purifying Compound or Resin]

The compound or the resin according to the present embodiment (i.e., the tannin compound according to the present embodiment) can be purified by the following method.

The purification method comprises the steps of:
obtaining a solution (S) by dissolving one or more tannin compounds according to the present embodiment in a solvent; and
extracting impurities in the compound or the resin by bringing the obtained solution (S) into contact with an acidic aqueous solution (a first extraction step), wherein
the solvent used in the step of obtaining the solution (S) contains an organic solvent that does not inadvertently mix with water.

In the first extraction step, the above resin is preferably a resin obtained by a reaction between the compound represented by the formula (0), the formula (1), the formula (0-1) or the formula (0-2), or the condensate having a structure derived from the compound represented by the formula (0-1) and/or the formula (0-2), and a crosslinking compound reactable with the compound or the condensate.

According to the purification method according to the present embodiment, the contents of various metals that may be contained as impurities in the compound or the resin having a specific structure described above can be reduced. In the present embodiment, the contents of metals that may be contained as impurities can be measured by ICP-MS analysis, and, for example, measurement equipment such as "ELAN DRC II" manufactured by PerkinElmer can be used.

More specifically, in the purification method according to the present embodiment, the above compound or resin is dissolved in an organic solvent that does not inadvertently mix with water to obtain the solution (S), and further, extraction treatment can be carried out by bringing the solution (S) into contact with an acidic aqueous solution. Thereby, metals contained in the solution (S) containing the tannin compound according to the present embodiment are transferred to the aqueous phase, then the organic phase and the aqueous phase are separated, and thus the tannin compound according to the present embodiment having a reduced metal content can be obtained.

The tannin compound according to the present embodiment used in the purification method according to the present embodiment may be alone as one kind, or may be a mixture of two or more kinds. Also, the tannin compound according to the present embodiment may contain various surfactants, various crosslinking agents, various acid generators, various stabilizers, and the like.

The organic solvent that does not inadvertently mix with water used in the purification method according to the present embodiment is not particularly limited, but is preferably an organic solvent that is safely applicable to semiconductor manufacturing processes, and specifically it is an organic solvent having a solubility in water at room temperature of less than 30%, and more preferably is an organic solvent having a solubility of less than 20% and particularly preferably less than 10%. The amount of the organic solvent used is preferably 1 to 100 parts by mass based on the tannin compound according to the present embodiment used.

Specific examples of the organic solvent that does not inadvertently mix with water include, but not limited to, organic solvents described in International Publication No. WO 2015/080240. Among these, toluene, 2-heptanone, cyclohexanone, cyclopentanone, methyl isobutyl ketone, propylene glycol monomethyl ether acetate, ethyl acetate, and the like are preferable, methyl isobutyl ketone, ethyl acetate, cyclohexanone, and propylene glycol monomethyl ether acetate are more preferable, and methyl isobutyl ketone and ethyl acetate are still preferable. Methyl isobutyl ketone, ethyl acetate, and the like have relatively high saturation solubility for the tannin compound according to the present embodiment and a relatively low boiling point, and it is thus possible to reduce the load in the case of industrially distilling off the solvent and in the step of removing the solvent by drying.

These organic solvents can be each used alone, and can be used as a mixture of two or more kinds.

The acidic aqueous solution used in the purification method according to the present embodiment can be arbitrarily selected from aqueous solutions in which generally known organic compounds or inorganic compounds are dissolved in water. Examples of the acidic aqueous solution include, but not limited to, acidic aqueous solutions described in International Publication No. WO 2015/080240. These acidic aqueous solutions can be each used alone, and can be also used as a combination of two or more kinds. Among these acidic aqueous solutions, aqueous solutions of one or more mineral acids selected from the group consisting of hydrochloric acid, sulfuric acid, nitric acid, and phosphoric acid, or aqueous solutions of one or more organic acids selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid, and trifluoroacetic acid are preferable, aqueous solutions of sulfuric acid, nitric acid, and carboxylic acids such as acetic acid, oxalic acid, tartaric acid, and citric acid are more preferable, aqueous solutions of sulfuric acid, oxalic acid, tartaric acid, and citric acid are still more preferable, and an aqueous solution of oxalic acid is particularly preferable. Polyvalent carboxylic acids such as oxalic acid, tartaric acid, and citric acid coordinate with metal ions and provide a chelating effect, and thus tend to be capable of more effectively removing metals. As for water used herein, it is preferable to use water, the metal content of which is small, such as ion exchanged water.

The pH of the acidic aqueous solution is not particularly limited, but it is preferable to regulate the acidity of the aqueous solution in consideration of an influence on the tannin compound according to the present embodiment. Normally, the pH range is about 0 to 5, and is preferably about pH 0 to 3.

The amount of the acidic aqueous solution is not particularly limited, but it is preferable to regulate the amount from the viewpoint of reducing the number of extraction operations for removing metals and from the viewpoint of ensuring operability in consideration of the overall amount of fluid. The amount of the acidic aqueous solution used is preferably 10 to 200% by mass, more preferably 20 to 100% by mass, based on 100% by mass of the solution (S).

In the purification method of the present embodiment, by bringing an acidic aqueous solution into contact with the solution (S) containing the tannin compound according to the present embodiment and the organic solvent that does not inadvertently mix with water, metals can be extracted from the tannin compound according to the present embodiment in the solution (S).

In the purification method of the present embodiment, it is preferable that the solution (S) further contains an organic solvent that inadvertently mixes with water. When the organic solvent that inadvertently mixes with water is contained, there is a tendency that the amount of the tannin compound according to the present embodiment charged can be increased, also the fluid separability is improved, and purification can be carried out at a high reaction vessel efficiency. The method for adding the organic solvent that inadvertently mixes with water is not particularly limited. For example, any of a method involving adding it to the organic solvent-containing solution in advance, a method involving adding it to water or the acidic aqueous solution in advance, and a method involving adding it after bringing the organic solvent-containing solution into contact with water or the acidic aqueous solution. Among these, the method involving adding it to the organic solvent-containing solution in advance is preferable in terms of the workability of operations and the ease of managing the amount.

The organic solvent that inadvertently mixes with water is not particularly limited, but is preferably an organic solvent that is safely applicable to semiconductor manufacturing processes. The amount of the organic solvent used that inadvertently mixes with water is not particularly limited as long as the solution phase and the aqueous phase separate, but is preferably 0.1 to 100 times, more preferably 0.1 to 50 times, and further preferably 0.1 to 20 times the mass of the tannin compound according to the present embodiment.

Specific examples of the organic solvent that inadvertently mixes with water include, but not particularly limited to, organic solvents described in International Publication No. WO 2015/080240. Among these, N-methylpyrrolidone, propylene glycol monomethyl ether, and the like are preferable, and N-methylpyrrolidone and propylene glycol monomethyl ether are more preferable. These solvents can be each used alone, and can be used as a mixture of two or more kinds.

In the purification method of the present embodiment, the temperature when the solution (S) and the acidic aqueous solution are brought into contact, i.e., when extraction treatment is carried out, is preferably in the range of 20 to 90°C, and more preferably 30 to 80°C. The extraction operation is not particularly limited, and is carried out, for example, by thoroughly mixing the solution (S) and the acidic aqueous solution by stirring or the like and then leaving the obtained mixed solution to stand still. Thereby, metals contained in the solution (S) containing the tannin compound according to the present embodiment and the organic solvents are transferred to the aqueous phase. Also, by this operation, the acidity of the solution (S) is lowered, and the degradation of the tannin compound according to the present embodiment can be suppressed.

By being left to stand still, the mixed solution is separated into an aqueous phase and a solution phase containing the tannin compound according to the present embodiment and the organic solvents, and thus the solution phase containing the tannin compound according to the present embodiment and the organic solvents is recovered by decantation. The time to stand still is not particularly limited, but it is preferable to regulate the time to stand still from the viewpoint of attaining good separation of the solution phase containing the organic solvents and the aqueous phase. Normally, the time to stand still is 1 minute or longer, preferably 10 minutes or longer, and more preferably 30 minutes or longer. While the extraction treatment may be carried out once, it is effective to repeat mixing, leaving-to-stand-still, and separating operations multiple times.

It is preferable that the purification method according to the present embodiment includes the step of extracting impurities in the tannin compound according to the present embodiment by further bringing the solution phase containing the tannin compound according to the present embodiment into contact with water after the first extraction step (the second extraction step).

Specifically, for example, it is preferable that after the extraction treatment is carried out using an acidic aqueous solution, the solution phase that is extracted and recovered from the aqueous solution and that contains the tannin compound according to the present embodiment and the organic solvents is further subjected to extraction treatment with water. The extraction treatment with water is not particularly limited, and can be carried out, for example, by thoroughly mixing the solution phase and water by stirring or the like and then leaving the obtained mixed solution to stand still. The mixed solution after being left to stand still is separated into an aqueous phase and a solution phase containing the tannin compound according to the present embodiment and the organic solvents, and thus the solution phase containing the tannin compound according to the present embodiment and the organic solvents can be recovered by decantation.

Water used herein is preferably water, the metal content of which is small, such as ion exchanged water. While the extraction treatment may be carried out once, it is effective to repeat mixing, leaving-to-stand-still, and separating operations multiple times. The proportions of both used in the extraction treatment and temperature, time, and other conditions are not particularly limited, and may be the same as those of the previous contact treatment with the acidic aqueous solution.

Water that is possibly present in the thus-obtained solution containing the tannin compound according to the present embodiment and the organic solvents can be easily removed by performing vacuum distillation or a like operation. Also, if required, the concentration of the tannin compound according to the present embodiment can be regulated to be any concentration by adding an organic solvent to the solution.

The method for isolating the tannin compound according to the present embodiment from the obtained solution containing the tannin compound according to the present embodiment and the organic solvents is not particularly limited, and publicly known methods can be carried out, such as reduced-pressure removal, separation by reprecipitation, and a combination thereof. Also, publicly known treatments such as concentration operation, filtration operation, centrifugation operation, and drying operation can be carried out if required.

### [Physical Properties and the Like of Resist Composition]

The resist composition of the present embodiment can form an amorphous film by spin coating. Depending on the kind of a developing solution to be used, a positive type resist pattern and a negative type resist pattern can be individually prepared.

In the case of a positive type resist pattern, the dissolution rate of the amorphous film formed by spin coating with the resist composition of the present embodiment in a developing solution at 23°C is preferably 5 angstrom/sec or less, more preferably 0.05 to 5 angstrom/sec, and further preferably 0.0005 to 5 angstrom/sec. When the dissolution rate is 5 angstrom/sec or less, there is a tendency that the amorphous film is insoluble in a developing solution and easily forms a resist. When the amorphous film has a dissolution rate of 0.0005 angstrom/sec or more, the resolution tends to improve. It is presumed that this is because due to the change in the solubility before and after exposure of the tannin compound according to the present embodiment, contrast at the interface between the exposed portion being dissolved in a developing solution and the unexposed portion not being dissolved in a developing solution is increased. Also, there are effects of reducing LER and defects.

In the case of using a negative type resist pattern, the dissolution rate of the amorphous film formed by spin coating with the resist composition of the present embodiment in a developing solution at 23°C is preferably 10 angstrom/sec or more. When the dissolution rate is 10 angstrom/sec or more, the amorphous film more easily dissolves in a developing solution, and is more suitable for a resist. When the dissolution rate is 10 angstrom/sec or more, the resolution tends to improve. It is presumed that this is because the micro surface portion of the tannin compound according to the present embodiment dissolves, and LER is reduced. Also, there are effects of reducing defects.

The dissolution rate can be determined by immersing the amorphous film in a developing solution for a predetermined period of time at 23°C and then measuring the film thickness before and after immersion by a publicly known method such as visual, ellipsometric, or QCM method.

In the case of using a positive type resist pattern, the dissolution rate of the portion exposed by radiation such as KrF excimer laser, extreme ultraviolet, electron beam or X-ray, of the amorphous film formed by spin coating with the resist composition of the present embodiment, in a developing solution at 23°C is preferably 10 angstrom/sec or more. When the dissolution rate is 10 angstrom/sec or more, the amorphous film more easily dissolves in a developing solution, and is more suitable for a resist. When the dissolution rate is 10 angstrom/sec or more, the resolution tends to improve. It is presumed that this is because the micro surface portion of the tannin compound according to the present embodiment dissolves, and LER is reduced. Also, there are effects of reducing defects.

In the case of a negative type resist pattern, the dissolution rate of the portion exposed by radiation such as KrF excimer laser, extreme ultraviolet, electron beam or X-ray, of the amorphous film formed by spin coating with the resist composition of the present embodiment, in a developing solution at 23°C is preferably 5 angstrom/sec or less, more preferably 0.05 to 5 angstrom/sec, and further preferably 0.0005 to 5 angstrom/sec. When the dissolution rate is 5 angstrom/sec or less, there is a tendency that the amorphous film is insoluble in a developing solution and easily forms a resist. When the dissolution rate is 0.0005 angstrom/sec or more, the resolution tends to improve. It is presumed that this is because due to the change in the solubility before and after exposure of the tannin compound according to the present embodiment, contrast at the interface between the unexposed portion being dissolved in a developing solution and the exposed portion not being dissolved in a developing solution is increased. Also, there are effects of reducing LER and defects.

### [Other Components of Resist Composition]

The resist composition of the present embodiment contains the tannin compound according to the present embodiment as a solid component. That is, the resist composition of the present embodiment may contain, each alone or in combination, a compound selected from compounds represented by the formula (0), the formula (1), and the formula (0-1) or the formula (0-2), and a condensate of one or more selected from the group consisting of the compounds represented by the formula (0-1) and/or the formula (0-2), and a resin obtained using, as a monomer, the compound selected from the compounds represented by the formula (0), the formula (1), and the formula (0-1) or the formula (0-2), and the condensate of one or more selected from the group consisting of the compounds represented by the formula (0-1) and/or the formula (0-2).

It is preferable that the resist composition of the present embodiment further contains a solvent. Examples of the solvent include, but not particularly limited to, solvents described in International Publication No. WO 2013/024778.

The solvent contained in the resist composition of the present embodiment is preferably a safe solvent, more preferably at least one selected from propylene glycol monomethyl ether acetate (PGMEA), propylene glycol monomethyl ether (PGME), cyclohexanone (CHN), cyclopentanone (CPN), 2-heptanone, anisole, butyl acetate, ethyl propionate, and ethyl lactate, and still more preferably at least one selected from PGMEA, PGME, and CHN.

In the resist composition of the present embodiment, the ratio between the amount of the solid component and the amount of the solvent is not particularly limited, but preferably the solid component is 1 to 80% by mass and the solvent is 20 to 99% by mass, more preferably the solid component is 1 to 50% by mass and the solvent is 50 to 99% by mass, still more preferably the solid component is 2 to 40% by mass and the solvent is 60 to 98% by mass, and particularly preferably the solid component is 2 to 10% by mass and the solvent is 90 to 98% by mass, based on 100% by mass of the total mass of the amount of the solid component and the solvent.

The resist composition of the present embodiment may contain at least one selected from the group consisting of an acid generating agent (P), an acid diffusion controlling agent (E), and a further component (F), as other solid components.

In the resist composition of the present embodiment, the content of the tannin compound according to the present embodiment is not particularly limited, but is preferably 50 to 99.4% by mass of the total mass of the solid components (summation of the tannin compound according to the present embodiment, and optionally used solid components such as acid generating agent (P), acid diffusion controlling agent (E), and further component (F), hereinafter the same), more preferably 55 to 90% by mass, still more preferably 60 to 80% by mass, and particularly preferably 60 to 70% by mass. When the content of the tannin compound according to the present embodiment is within the above range, there is a tendency that resolution is further improved, and line edge roughness (LER) is further decreased.

When both the compound and the resin are contained as the tannin compound according to the present embodiment, the content refers to the total amount of the tannin compounds according to the present embodiment (i.e., the total amount of the compound and the resin).

### (Acid Generating Agent)

The resist composition of the present embodiment preferably contains one or more acid generating agents (P) generating an acid directly or indirectly by irradiation of any radiation selected from visible light, ultraviolet, excimer laser, electron beam, extreme ultraviolet (EUV), X-ray, and ion beam.

In this case, in the resist composition, the content of the acid generating agent (P) is preferably 0.001 to 49% by mass of the total mass of the solid components, more preferably 1 to 40% by mass, still more preferably 3 to 30% by mass, and particularly preferably 10 to 25% by mass. When the content of the acid generating agent (P) is within the above range, there is a tendency that a pattern profile with even higher sensitivity and even lower edge roughness is obtained.

Concerning the resist composition of the present embodiment, the acid generation method is not particularly limited as long as an acid is generated in the system. By using excimer laser instead of ultraviolet such as g-ray and i-ray, finer processing is possible, and also by using electron beam, extreme ultraviolet, X-ray or ion beam as a high energy ray, further finer processing is possible.

The acid generating agent (P) is not particularly limited, and an acid generating agent described in International Publication No. WO 2013/024778 can be used. Among these acid generating agents, an acid generating agent having an aromatic ring is preferable, and an acid generating agent represented by the following formula (8-1) or (8-2) is more preferable, from the viewpoint of heat resistance: 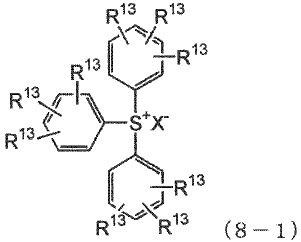 (In the formula (8-1), R¹³ may be the same or different, and are each independently a hydrogen atom, a linear, branched or cyclic alkyl group, a linear, branched or cyclic alkoxy group, a hydroxyl group, or a halogen atom, X⁻ is an alkyl group, an aryl group, a sulfonic acid ion having a halogen substituted alkyl group or a halogen substituted aryl group, or a halide ion.) 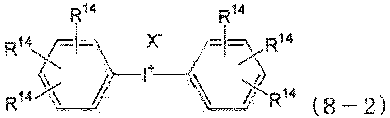 (In the formula (8-2), R¹⁴ may be the same or different, and each independently represents a hydrogen atom, a linear, branched or cyclic alkyl group, a linear, branched or cyclic alkoxy group, a hydroxyl group, or a halogen atom. X⁻ is the same as above.)

An acid generating agent having a sulfonate ion wherein X⁻ of the above formula (8-1) or (8-2) has an aryl group or a halogen-substituted aryl group is further preferable; an acid generating agent having a sulfonate ion wherein X⁻ of the formula (8-1) or (8-2) has an aryl group is still more preferable; and diphenyltrimethylphenylsulfonium p-toluenesulfonate, triphenylsulfonium p-toluenesulfonate, triphenylsulfonium trifluoromethanesulfonate, and triphenylsulfonium nonafluoromethanesulfonate are particularly preferable. By using the acid generating agent, there is a tendency that LER can be reduced.

The acid generating agent (P) can be used alone as one kind or in combination of two or more kinds.

### (Acid Diffusion Controlling Agent)

The resist composition of the present embodiment may contain an acid diffusion controlling agent (E) having a function of controlling diffusion of an acid generated from an acid generating agent by radiation irradiation in a resist film to inhibit any unpreferable chemical reaction in an unexposed region or the like. By using the acid diffusion controlling agent (E), the storage stability of a resist composition is improved. Also, along with the further improvement of the resolution, the line width change of a resist pattern due to variation in the post exposure delay time before radiation irradiation and the post exposure delay time after radiation irradiation can be inhibited, and the composition has extremely excellent process stability.

The acid diffusion controlling agent (E) is not particularly limited, and examples include a radiation degradable basic compound such as a nitrogen atom-containing basic compound, a basic sulfonium compound, and a basic iodonium compound described in International Publication No. WO 2013/024778. The acid diffusion controlling agent (E) can be used alone as one kind or in combination of two or more kinds.

The content of the acid diffusion controlling agent (E) is preferably 0.001 to 49% by mass of the total mass of the solid component, more preferably 0.01 to 10% by mass, still more preferably 0.01 to 5% by mass, and particularly preferably 0.01 to 3% by mass. When the content of the acid diffusion controlling agent (E) is within the above range, there is a tendency that a decrease in resolution, and deterioration of the pattern shape and the dimension fidelity or the like can be further inhibited. Moreover, even though the post exposure delay time from electron beam irradiation to heating after radiation irradiation becomes longer, the shape of the pattern upper layer portion is less likely to deteriorate. When the content of the acid diffusion controlling agent (E) is 10% by mass or less, there is a tendency that a decrease in sensitivity, and developability of the unexposed portion or the like can be prevented. By using such an acid diffusion controlling agent, the storage stability of a resist composition improves, also along with improvement of the resolution, the line width change of a resist pattern due to variation in the post exposure delay time before radiation irradiation and the post exposure delay time after radiation irradiation can be inhibited, and the composition is extremely excellent process stability.

To the resist composition of the present embodiment, within the range of not inhibiting the purpose of the present invention, if required, as the other component (F), one kind or more of various additive agents such as a dissolution promoting agent, a dissolution controlling agent, a sensitizing agent, an acid crosslinking agent, a surfactant and an organic carboxylic acid or an oxo acid of phosphor, or derivative thereof can be added.

### (Dissolution Promoting Agent)

The dissolution promoting agent is a component having a function of increasing the solubility of the tannin compound according to the present embodiment in a developing solution to moderately increase the dissolution rate of the tannin compound according to the present embodiment upon developing, when the solubility of the tannin compound is too low. The low molecular weight dissolution promoting agent can be used, within the range of not deteriorating the effect of the present invention. Examples of the above dissolution promoting agent can include low molecular weight phenolic compounds, such as bisphenols and tris(hydroxyphenyl)methane. These dissolution promoting agents can be used alone as one kind or in mixture of two or more kinds. The content of the dissolution promoting agent, which is arbitrarily adjusted according to the kind of the tannin compound according to the present embodiment to be used, is preferably 0 to 49% by mass of the total mass of the solid component, more preferably 0 to 5% by mass, still more preferably 0 to 1% by mass, and particularly preferably 0% by mass.

### (Dissolution Controlling Agent)

The dissolution controlling agent is a component having a function of controlling the solubility of the tannin compound according to the present embodiment in a developing solution to moderately decrease the dissolution rate upon developing, when the solubility of the tannin compound is too high. As such a dissolution controlling agent, the one which does not chemically change in steps such as calcination of resist coating, radiation irradiation, and development is preferable.

The dissolution controlling agent is not particularly limited, and examples can include aromatic hydrocarbons such as phenanthrene, anthracene, and acenaphthene; ketones such as acetophenone, benzophenone, and phenyl naphtyl ketone; and sulfones such as methyl phenyl sulfone, diphenyl sulfone, and dinaphthyl sulfone. These dissolution controlling agents can be used alone as one kind or in two or more kinds.

The content of the dissolution controlling agent is not particularly limited and is arbitrarily adjusted according to the kind of the tannin compound according to the present embodiment to be used, but is preferably 0 to 49% by mass of the total mass of the solid component, more preferably 0 to 5% by mass, still more preferably 0 to 1% by mass, and particularly preferably 0% by mass.

### (Sensitizing Agent)

The sensitizing agent is a component having a function of absorbing irradiated radiation energy, transmitting the energy to the acid generating agent (P), and thereby increasing the acid production amount, and improving the apparent sensitivity of a resist. Such a sensitizing agent is not particularly limited, and examples can include benzophenones, biacetyls, pyrenes, phenothiazines, and fluorenes. These sensitizing agents can be used alone as one kind or in two or more kinds. The content of the sensitizing agent, which is arbitrarily adjusted according to the kind of the tannin compound according to the present embodiment to be used, is preferably 0 to 49% by mass of the total mass of the solid component, more preferably 0 to 5% by mass, still more preferably 0 to 1% by mass, and particularly preferably 0% by mass.

### (Acid crosslinking agent)

The acid crosslinking agent is a compound capable of intramolecular or intermolecular crosslinking the tannin compound according to the present embodiment in the presence of the acid generated from the acid generating agent (P). Such an acid crosslinking agent is not particularly limited, and, for example, an acid crosslinking agent described in International Publication No. WO 2013/024778 can be used. The acid crosslinking agent can be used alone or in combination of two or more kinds. The content of the acid crosslinking agent is preferably 0 to 49% by mass of the total mass of the solid component, more preferably 0 to 5% by mass, still more preferably 0 to 1% by mass, and particularly preferably 0% by mass.

### (Surfactant)

The surfactant is a component having a function of improving coatability and striation of the resist composition of the present embodiment, and developability of a resist or the like. The surfactant is not particularly limited, and may be any of anionic, cationic, nonionic or amphoteric. Among them, a nonionic surfactant is preferable. The nonionic surfactant has a good affinity with a solvent used in production of resist compositions, and the above effect is more marked. Examples of the nonionic surfactant include nonionic surfactants described in International Publication No. WO 2013/024778. The content of the surfactant is not particularly limited, and is arbitrarily adjusted according to the kind of the tannin compound according to the present embodiment to be used, but is preferably 0 to 49% by mass of the total mass of the solid component, more preferably 0 to 5% by mass, still more preferably 0 to 1% by mass, and particularly preferably 0% by mass.

### (Organic Carboxylic Acid or Oxo Acid of Phosphor or Derivative Thereof)

For the purpose of prevention of sensitivity deterioration or improvement of a resist pattern shape and post exposure delay stability or the like, and as an additional optional component, the resist composition of the present embodiment may contain an organic carboxylic acid or an oxo acid of phosphor or derivative thereof. These components can be used in combination with the acid diffusion controlling agent, or may be used alone. The organic carboxylic acid is not particularly limited, and, for example, is suitably malonic acid, citric acid, malic acid, succinic acid, benzoic acid, salicylic acid, or the like. Examples of the oxo acid of phosphor or derivative thereof include phosphoric acid or derivative thereof such as ester including phosphoric acid, di-n-butyl ester phosphate, and diphenyl ester phosphate; phosphonic acid or derivative thereof such as ester including phosphonic acid, dimethyl ester phosphonate, di-n-butyl ester phosphonate, phenylphosphonic acid, diphenyl ester phosphonate, and dibenzyl ester phosphonate; and phosphinic acid and derivative thereof such as ester including phosphinic acid and phenylphosphinic acid. Among these, phosphonic acid is particularly preferable.

The organic carboxylic acid or the oxo acid of phosphor or derivative thereof can be used alone as one kind or in combination of two or more kinds. The content of the organic carboxylic acid or the oxo acid of phosphor or derivative thereof, which is arbitrarily adjusted according to the kind of the tannin compound according to the present embodiment to be used, is preferably 0 to 49% by mass of the total mass of the solid component, more preferably 0 to 5% by mass, still more preferably 0 to 1% by mass, and particularly preferably 0% by mass.

### (Other Additive Agents)

The resist composition of the present embodiment may contain one kind or more of additive agents other than the above dissolution controlling agent, sensitizing agent, and surfactant, within the range of not inhibiting the purpose of the present invention, if required. Examples of such an additive agent include, but not particularly limited to, a dye, a pigment, and an adhesion aid. For example, the composition contains the dye or the pigment, and thereby a latent image of the exposed portion is visualized and influence of halation upon exposure can be alleviated, which is preferable. The composition contains the adhesion aid, and thereby adhesiveness to a substrate can be improved, which is preferable. Furthermore, examples of other additive agent can include, but not particularly limited to, a halation preventing agent, a storage stabilizing agent, a defoaming agent, and a shape improving agent. Specific examples thereof can include 4-hydroxy-4'-methylchalkone.

The total content of the optional component (F) is preferably 0 to 49% by mass of the total mass of the solid component, more preferably 0 to 5% by mass, still more preferably 0 to 1% by mass, and particularly preferably 0% by mass.

In the resist composition of the present embodiment, the content of the tannin compound according to the present embodiment, the acid generating agent (P), the acid diffusion controlling agent (E), the optional component (F) (the tannin compound according to the present embodiment/the acid generating agent (P)/the acid diffusion controlling agent (E)/the optional component (F)) is preferably 50 to 99.4/0.001 to 49/0.001 to 49/0 to 49 in % by mass based on the solid content, more preferably 55 to 90/1 to 40/0.01 to 10/0 to 5, still more preferably 60 to 80/3 to 30/0.01 to 5/0 to 1, and particularly preferably 60 to 70/10 to 25/0.01 to 3/0.

The content ratio of each component is selected from each range so that the summation thereof is 100% by mass. When the content ratio of each component is within the above range, performance such as sensitivity, resolution, and developability tends to be even better.

The method for preparing the resist composition of the present embodiment is not particularly limited, and, examples include a method involving dissolving each component in a solvent upon use into a homogenous solution, and then if required, filtering through a filter or the like with a pore diameter of about 0.2 µm, for example.

The resist composition of the present embodiment can contain various resins within the range of not inhibiting the purpose of the present invention. Examples of the various resins include, but not particularly limited to, a novolac resin, polyvinyl phenols, polyacrylic acid, polyvinyl alcohol, a styrene-maleic anhydride resin, an acrylic acid, vinyl alcohol or vinylphenol as a monomeric unit, or derivative thereof. The content of the resin is not particularly limited, and is arbitrarily adjusted according to the kind of the tannin compound according to the present embodiment to be used, but is preferably 30 parts by mass or less per 100 parts by mass of the compound or the resin, more preferably 10 parts by mass or less, still more preferably 5 parts by mass or less, particularly preferably 0 parts by mass.

### [Resist Pattern Formation Method]

The resist pattern formation method of the present embodiment is not particularly limited, and examples of a suitable method include a method comprising the steps of: forming a resist film on a substrate using the resist composition mentioned above; exposing the formed resist film; and developing the exposed film, thereby forming a resist pattern.

The resist pattern according to the present embodiment can also be formed as an upper layer resist in a multilayer process.

Specific examples of the resist pattern formation method include, but not particularly limited to, the following methods. A resist film is formed by coating a conventionally publically known substrate with the above resist composition using a coating means such as spin coating, flow casting coating, and roll coating. The conventionally publically known substrate is not particularly limited. For example, a substrate for electronic components, and the one having a predetermined wiring pattern formed thereon, or the like can be exemplified. More specific examples are not particularly limited, and examples include a substrate made of a metal such as a silicon wafer, copper, chromium, iron and aluminum, and a glass substrate. Examples of a wiring pattern material include, but not particularly limited to, copper, aluminum, nickel, and gold. Also if required, the substrate may be a substrate having an inorganic film and/or organic film provided thereon. Examples of the inorganic film include, but not particularly limited to, an inorganic antireflection film (inorganic BARC). Examples of the organic film include, but not particularly limited to, an organic antireflection film (organic BARC). The substrate may be subjected to surface treatment with hexamethylene disilazane or the like.

Next, the substrate coated with the resist composition is heated if required. The heating conditions vary according to the compounding composition of the resist composition, or the like, but are preferably 20 to 250°C, and more preferably 20 to 150°C. By heating the substrate, the adhesiveness of a resist to a substrate tends to improve, which is preferable. Then, the resist film is exposed to a desired pattern by any radiation selected from the group consisting of visible light, ultraviolet, excimer laser, electron beam, extreme ultraviolet (EUV), X-ray, and ion beam. The exposure conditions or the like are arbitrarily selected according to the compounding composition of the resist composition, or the like.

In the resist pattern formation method of the present embodiment, in order to stably form a fine pattern with a high degree of accuracy in exposure, the resist film is preferably heated after radiation irradiation. The heating conditions vary according to the compounding composition of the resist composition, or the like, but are preferably 20 to 250°C, and more preferably 20 to 150°C.

Next, by developing the exposed resist film in a developing solution, a predetermined resist pattern is formed.

As the developing solution, a solvent having a solubility parameter (SP value) close to that of the tannin compound according to the present embodiment to be used is preferably selected. A polar solvent such as a ketone-based solvent, an ester-based solvent, an alcohol-based solvent, an amide-based solvent, and an ether-based solvent; and a hydrocarbon-based solvent, or an alkaline aqueous solution can be used. For example, a solvent described in International Publication No. WO 2013/024778 can be used.

Depending on the kind of the developing solution, a positive type resist pattern and a negative type resist pattern can be individually prepared. In general, in the case of a polar solvent such as a ketone-based solvent, an ester-based solvent, an alcohol-based solvent, an amide-based solvent, and an ether-based solvent, or a hydrocarbon-based solvent, a negative type resist pattern is obtained, and in the case of an alkaline aqueous solution, a positive type resist pattern is obtained.

A plurality of above solvents may be mixed, or the solvent may be used by mixing the solvent with a solvent other than those described above or water within the range having performance. In order to sufficiently exhibit the effect of the present invention, the water content ratio as the whole developing solution is preferably less than 70% by mass, more preferably less than 50% by mass, still more preferably less than 30% by mass, and further preferably less than 10% by mass. Particularly preferably, the developing solution is substantially moisture free. That is, the content of the organic solvent in the developing solution is not particularly limited, and is preferably 30% by mass or more and 100% by mass or less based on the total amount of the developing solution, more preferably 50% by mass or more and 100% by mass or less, still more preferably 70% by mass or more and 100% by mass or less, further more preferably 90% by mass or more and 100% by mass or less, and particularly preferably 95% by mass or more and 100% by mass or less.

The alkaline aqueous solution is not particularly limited, and examples include an alkaline compound such as mono-, di- or tri-alkylamines, mono-, di- or trialkanolamines, heterocyclic amines, tetramethyl ammonium hydroxide (TMAH), and choline.

Particularly, the developing solution containing at least one kind of solvent selected from a ketone-based solvent, an ester-based solvent, an alcohol-based solvent, an amide-based solvent, and an ether-based solvent tends to further improve resist performance such as resolution and roughness of the resist pattern, which is preferable.

The vapor pressure of the developing solution is not particularly limited, and is preferably 5 kPa or less at 20°C, more preferably 3 kPa or less, and still more preferably 2 kPa or less, for example. There is a tendency that the evaporation of the developing solution on the substrate or in a developing cup is inhibited by setting the vapor pressure of the developing solution to 5 kPa or less, to improve temperature uniformity within a wafer surface, thereby resulting in improvement in size uniformity within the wafer surface.

Specific examples of the developing solution having a vapor pressure of 5 kPa or less include developing solutions described in International Publication No. WO 2013/024778.

Specific examples of the developing solution having a vapor pressure of 2 kPa or less which is a particularly preferable range include developing solutions described in International Publication No. WO 2013/024778.

To the developing solution, a surfactant can be added in an appropriate amount, if required. The surfactant is not particularly limited but, for example, an ionic or nonionic fluorine-based and/or silicon-based surfactant can be used. Examples of the fluorine-based and/or silicon-based surfactant include the surfactants described in Japanese Patent Application Laid-Open Nos. 62-36663, 61-226746, 61-226745, 62-170950, 63-34540, 7-230165, 8-62834, 9-54432, and 9-5988, and U.S. Pat. Nos. 5,405,720, 5,360,692, 5,529,881, 5,296,330, 5,436,098, 5,576,143, 5,294,511, and 5,824,451. The surfactant is preferably a nonionic surfactant. The nonionic surfactant is not particularly limited, but a fluorine-based surfactant or a silicon-based surfactant is further preferably used.

The amount of the surfactant used is usually 0.001 to 5% by mass based on the total amount of the developing solution, preferably 0.005 to 2% by mass, and further preferably 0.01 to 0.5% by mass.

The development method is, for example, a method for dipping a substrate in a bath filled with a developing solution for a fixed time (dipping method), a method for raising a developing solution on a substrate surface by the effect of a surface tension and keeping it still for a fixed time, thereby conducting the development (puddle method), a method for spraying a developing solution on a substrate surface (spraying method), and a method for continuously ejecting a developing solution on a substrate rotating at a constant speed while scanning a developing solution ejecting nozzle at a constant rate (dynamic dispense method), or the like may be applied. The time for conducting the pattern development is not particularly limited, but is preferably 10 seconds to 90 seconds.

After the step of conducting development, a step of stopping the development by the replacement with another solvent may be practiced.

A step of rinsing the resist film with a rinsing solution containing an organic solvent is preferably provided after the development.

The rinsing solution used in the rinsing step after development is not particularly limited as long as the rinsing solution does not dissolve the resist pattern cured by crosslinking. A solution containing a general organic solvent or water may be used as the rinsing solution. As the rinsing solution, a rinsing solution containing at least one kind of organic solvent selected from a hydrocarbon-based solvent, a ketone-based solvent, an ester-based solvent, an alcohol-based solvent, an amide-based solvent, and an ether-based solvent is preferably used. More preferably, after development, a step of rinsing the film by using a rinsing solution containing at least one kind of organic solvent selected from the group consisting of a ketone-based solvent, an ester-based solvent, an alcohol-based solvent and an amide-based solvent is conducted. Still more preferably, after development, a step of rinsing the film by using a rinsing solution containing an alcohol-based solvent or an ester-based solvent is conducted. Furthermore preferably, after development, a step of rinsing the film by using a rinsing solution containing a monohydric alcohol is conducted. Particularly preferably, after development, a step of rinsing the film by using a rinsing solution containing a monohydric alcohol having 5 or more carbon atoms is conducted. The time for rinsing the pattern is not particularly limited, but is preferably 10 seconds to 90 seconds.

Herein, examples of the monohydric alcohol used in the rinsing step after development are not particularly limited, and specific examples include monohydric alcohols described in International Publication No. WO 2013/024778.

A plurality of these components may be mixed, or the component may be used by mixing the component with an organic solvent other than those described above.

The water content ratio in the rinsing solution is not particularly limited, and is preferably 10% by mass or less, more preferably 5% by mass or less, and particularly preferably 3% by mass or less. By setting the water content ratio in the rinsing solution to 10% by mass or less, there is a tendency that better development characteristics can be obtained.

The vapor pressure at 20°C of the rinsing solution used after development is preferably 0.05 kPa or more and 5 kPa or less, more preferably 0.1 kPa or more and 5 kPa or less, and much more preferably 0.12 kPa or more and 3 kPa or less. By setting the vapor pressure of the rinsing solution to 0.05 kPa or more and 5 kPa or less, there is a tendency that the temperature uniformity in the wafer surface is enhanced and moreover, swelling due to permeation of the rinsing solution is further inhibited. As a result, the dimensional uniformity in the wafer surface tends to be further improved.

The rinsing solution may also be used after adding an appropriate amount of a surfactant to the rinsing solution.

In the rinsing step, the wafer after development is rinsed using the organic solvent-containing rinsing solution. The method for rinsing treatment is not particularly limited. However, for example, a method for continuously ejecting a rinsing solution on a substrate spinning at a constant speed (spin coating method), a method for dipping a substrate in a bath filled with a rinsing solution for a fixed time (dipping method), and a method for spraying a rinsing solution on a substrate surface (spraying method), or the like can be applied. Above all, it is preferable to conduct the rinsing treatment by the spin coating method and after the rinsing, spin the substrate at a rotational speed of 2,000 rpm to 4,000 rpm, to remove the rinsing solution from the substrate surface.

After forming the resist pattern, a pattern wiring substrate is obtained by etching. Etching can be conducted by a publicly known method such as dry etching using plasma gas, and wet etching with an alkaline solution, a cupric chloride solution, and a ferric chloride solution or the like.

After forming the resist pattern, plating can also be conducted. Examples of the above plating method include, but not particularly limited to, copper plating, solder plating, nickel plating, and gold plating.

The remaining resist pattern after etching can be peeled by an organic solvent. Examples of the above organic solvent are not particularly limited, and examples include PGMEA (propylene glycol monomethyl ether acetate), PGME (propylene glycol monomethyl ether), and EL (ethyl lactate). Examples of the above peeling method are not particularly limited, and examples include a dipping method and a spraying method. A wiring substrate having a resist pattern formed thereon may be a multilayer wiring substrate, and may have a small diameter through hole.

In the present embodiment, the wiring substrate can also be formed by a method for forming a resist pattern, then depositing a metal in vacuum, and subsequently dissolving the resist pattern in a solution, i.e., a liftoff method.

### Examples

The present invention will be more specifically described with reference to examples below. However, the present invention is not limited to these examples.

Below, methods for measuring a compound and methods for evaluating resist performance and the like in examples are presented.

### [Measurement Method]

### (1) Structure of Compound

The structure of the compound was verified by carrying out ¹H-NMR measurement under the following conditions using "Advance 600 II spectrometer" manufactured by Bruker.
Frequency: 400 MHz
Solvent: d6-DMSO
Internal standard: TMS
Measurement temperature: 23°C

### [Evaluation Method]

### (1) Safe Solvent Solubility Test of Compound

The solubility of the compound in propylene glycol monomethyl ether acetate (PGMEA) was evaluated according to the following criteria utilizing the amount of dissolution in PGMEA. The amount of dissolution was measured at 23°C by precisely weighing the compound into a test tube, adding PGMEA so as to attain a predetermined concentration, applying ultrasonic waves for 30 minutes in an ultrasonic cleaner, and then visually observing the subsequent state of the fluid. The evaluation was conducted according to the following.
A: 5.0% by mass ≤ Amount of dissolution
B: 3.0% by mass ≤ Amount of dissolution < 5.0% by mass
C: Amount of dissolution < 3.0% by mass

### (2) Storage Stability and Thin Film Formability of Resist Composition

The storage stability of a resist composition was evaluated by leaving the resist composition to stand still for three days at 23°C and 50% RH after preparation and then visually observing the resist composition for the presence and absence of precipitates. The resist composition after being left to stand still for three days was evaluated as "A" when it was a homogeneous solution without precipitates, and "C" when precipitates were present. A clean silicon wafer was spin coated with the resist composition in a homogeneous state, and then prebaked before exposure in an oven of 110°C to form a resist film with a thickness of 40 nm. The prepared resist composition was evaluated as "A" when the thin film formability was good, and "C" when the formed film had defects.

### (3) Pattern Evaluation of Resist Pattern

A clean silicon wafer was spin coated with a homogeneous resist composition, and then prebaked before exposure in an oven of 110°C to form a resist film with a thickness of 60 nm. The obtained resist film was irradiated with electron beams of 1:1 line and space setting with 50 nm, 40 nm, and 30 nm intervals using an electron beam lithography system (ELS-7500 manufactured by ELIONIX INC.). After irradiation, the resist film was heated at each predetermined temperature for 90 seconds, and immersed in 2.38% by mass TMAH alkaline developing solution for 60 seconds for development. Subsequently, the resist film was washed with ultrapure water for 30 seconds, and dried to form a positive type resist pattern. Concerning the formed resist pattern, the line and space were observed using a scanning electron microscope ("S-4800" manufactured by Hitachi High-Technologies Corporation) to evaluate the reactivity by electron beam irradiation of the resist composition.

The "sensitivity" was indicated by the minimum amount of energy per unit area necessary for obtaining the pattern, and evaluated according to the following. A: when the pattern was obtained at less than 50 µC/cm² C: when the pattern was obtained at 50 µC/cm² or more.

The "pattern formability" was evaluated according to the following by observing the obtained pattern shape under SEM (scanning electron microscope).
A: when a rectangular pattern was obtained
B: when an almost rectangular pattern was obtained
C: when a non-rectangular pattern was obtained

### [Synthesis Examples]

### (Synthesis Example 1) Synthesis of TNA-ADBAC

In a container (internal capacity: 200 mL) equipped with a stirrer, a condenser tube, and a burette, 3.9 g (2.3 mmol) of tannic acid (TNA), 0.30 g (22 mmol) of potassium carbonate, and 0.64 g (2 mmol) of tetrabutyl ammonium bromide were dissolved in 50 mL of N-methylpyrrolidone, and the solution was stirred for 2 hours. After stirring, 13.2 g (46 mmol) of bromoacetic acid-2-methyladamantan-2-yl was added thereto, and the mixture was reacted at 100°C for 24 hours. After the reaction terminated, the reaction mixture was dropped to a 1 N aqueous hydrochloric acid solution, and the resulting black solid was filtered off and separated and purified by column chromatography to obtain 7.8 g of the objective compound represented by the following formula (TNA-ADBAC).

The following peaks were found by NMR measurement performed on the obtained compound (TNA-ADBAC) under the above measurement conditions, and the compound was confirmed to have a chemical structure of the following formula (TNA-ADBAC).
δ (ppm) 6.5-7.5 (20H, Ph-H), 4.4-5.0 (37H, O-CH₂-C(=O)-), 1.2-2.7 (314.5H, C-H/Adamantane of methylene and methine), 3.9-4.0 (4H, C-CH(-O)-C), 5.4 (1H, O-CH(-O)-), 9.3-9.6 (6.5H, -OH)

As a result of measuring the introduction rate of the acid dissociation reactive group in the obtained compound (TNA-ADBAC) by NMR, it was 74%.

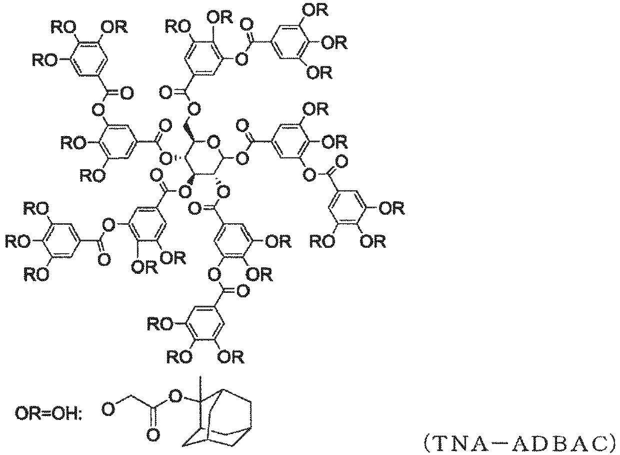

Also, the obtained compound (TNA-ADBAC) was evaluated for its solubility in a safe solvent by the above method. The results are shown in Table 1 (Examples 1 and 1-2).

### (Synthesis Example 2) Synthesis of TNA-ADBAC100

In a container (internal capacity: 100 mL) equipped with a stirrer, a condenser tube, and a burette, 1.7 g (1 mmol) of tannic acid (TNA), 3.46 g (25 mmol) of potassium carbonate, and 2.437 g (7.56 mmol) of tetrabutyl ammonium bromide were dissolved in 25 mL of N-methylpyrrolidone, and the solution was stirred for 2 hours. After stirring, 9.01 g (31.4 mmol) of bromoacetic acid-2-methyladamantan-2-yl was added thereto, and the mixture was reacted at 100°C for 24 hours. After the reaction terminated, the reaction mixture was dropped to a 1 N aqueous hydrochloric acid solution, and the resulting black solid was filtered off and separated and purified by column chromatography to obtain 1.01 g of the objective compound represented by the following formula (TNA-ADBAC100).

The following peaks were found by NMR measurement performed on the obtained compound (TNA-ADBAC100) under the above measurement conditions, and the compound was confirmed to have a chemical structure of the following formula (TNA-ADBAC100).

δ (ppm) 6.5-7.5 (20H, Ph-H), 4.4-5.0 (50H, O-CH₂-C(=O)-), 1.2-2.7 (425H, C-H/Adamantane of metyl, methylene and methine), 3.9-4.0 (4H, C-CH(-O)-C), 5.4 (1H, O-CH(-O)-)

As a result of measuring the introduction rate of the acid dissociation reactive group in the obtained compound (TNA-ADBAC100) by NMR, it was 100%.

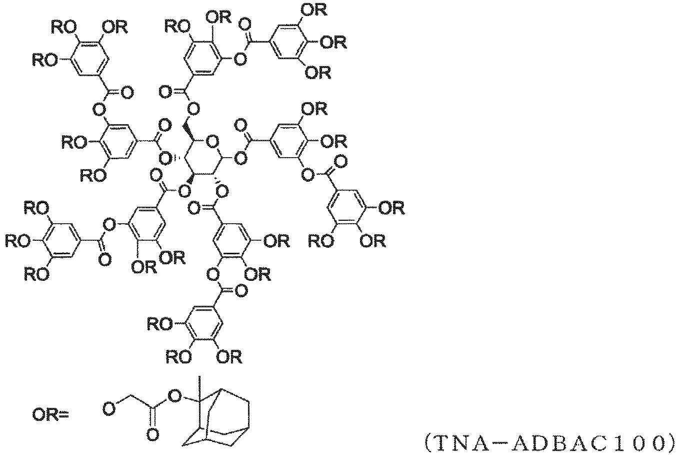

Also, the obtained compound (TNA-ADBAC100) was evaluated for its solubility in a safe solvent by the above method. The results are shown in Table 1 (Example 2) .

### (Synthesis Example 3) Synthesis of TNA-BVE

The same synthesis as in Synthesis Example 1 was performed except that butyl vinyl ether was used in place of bromoacetic acid-2-methyladamantan-2-yl, and tetrahydrofuran was used in place of N-methylpyrrolidone as a solvent, to obtain the objective compound having a chemical structure of the following formula (TNA-BVE).

The following peaks were found by NMR measurement performed on the obtained compound under the above measurement conditions, and the compound was confirmed to have a chemical structure of the following formula (TNA-BVE).
δ (ppm) 6.5-7.5 (20H, Ph-H), 4.4-5.0 (37H, O-CH₂-C(=O)-), 5.5 (24H, C-H/BVE of methine), 1.0-3.4 (288H, C-H/BVE of methyl and methylene), 3.9-4.0 (4H, C-CH(-O)-C), 5.4 (1H, O-CH(-O)-), 9.3-9.6 (1H, -OH)

As a result of measuring the introduction rate of the acid dissociation reactive group in the obtained compound (TNA-BVE) by NMR, it was 96%.

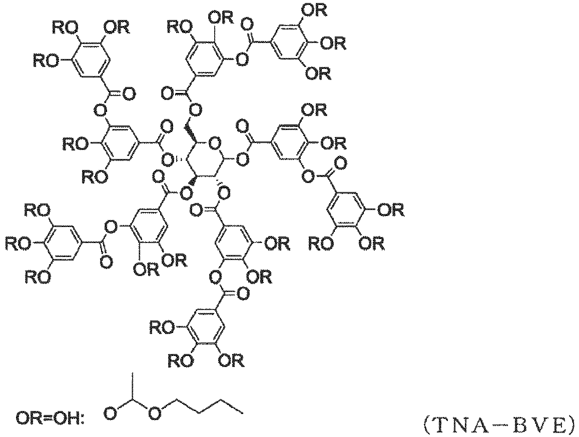

Also, the obtained compound (TNA-BVE) was evaluated for its solubility in a safe solvent by the above method. The results are shown in Table 1 (Example 3).

### (Synthesis Example 4) Synthesis of TNA-BOC

The same synthesis as in Synthesis Example 1 was performed except that di-t-butyl dicarbonate was used in place of bromoacetic acid-2-methyladamantan-2-yl, and tetrahydrofuran was used in place of N-methylpyrrolidone as a solvent, to obtain the objective compound having a chemical structure of the following formula (TNA-BOC).

The following peaks were found by NMR measurement performed on the obtained compound under the above measurement conditions, and the compound was confirmed to have a chemical structure of the following formula (TNA-BOC).
δ (ppm) 6.5-7.5 (20H, Ph-H), 4.4-5.0 (37H, O-CH₂-C(=O)-), 1.4 (225H, C-H/BOC of methyl), 3.9-4.0 (4H, C-CH(-O)-C), 5.4 (1H, O-CH(-O)-)

As a result of measuring the introduction rate of the acid dissociation reactive group in the obtained compound (TNA-BOC) by NMR, it was 100%.

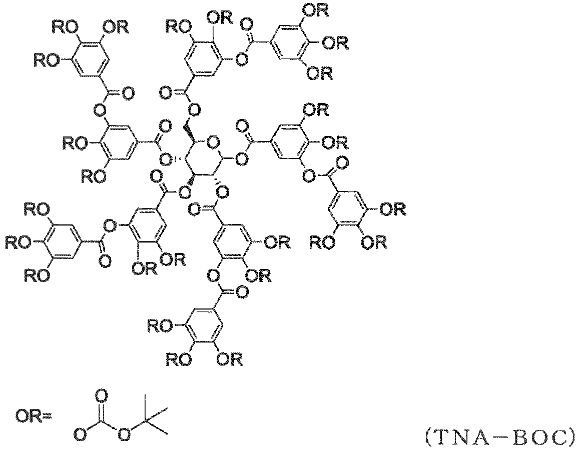

Also, the obtained compound (TNA-BOC) was evaluated for its solubility in a safe solvent by the above method. The results are shown in Table 1 (Example 4).

### (Synthesis Example 5) Synthesis of TNA-MeBOC

The same synthesis as in Synthesis Example 1 was performed except that t-butyl bromoacetate was used in place of bromoacetic acid-2-methyladamantan-2-yl, tetrahydrofuran was used in place of N-methylpyrrolidone as a solvent, and 0.4 g (1.5 mmol) of 18-crown-6 was further added, to obtain the objective compound having a chemical structure of the following formula (TNA-MeBOC).

The following peaks were found by NMR measurement performed on the obtained compound under the above measurement conditions, and the compound was confirmed to have a chemical structure of the following formula (TNA-MeBOC) .
δ (ppm) 6.5-7.5 (20H, Ph-H), 4.4-5.0 (37H, O-CH₂-C(=O)-), 1.4 (225H, C-H/BOC of methyl), 4.5 (225H, C-H/BOC of methylene), 3.9-4.0 (4H, C-CH(-O)-C), 5.4 (1H, O-CH(-O)-)

As a result of measuring the introduction rate of the acid dissociation reactive group in the obtained compound (TNA-MeBOC) by NMR, it was 100%.

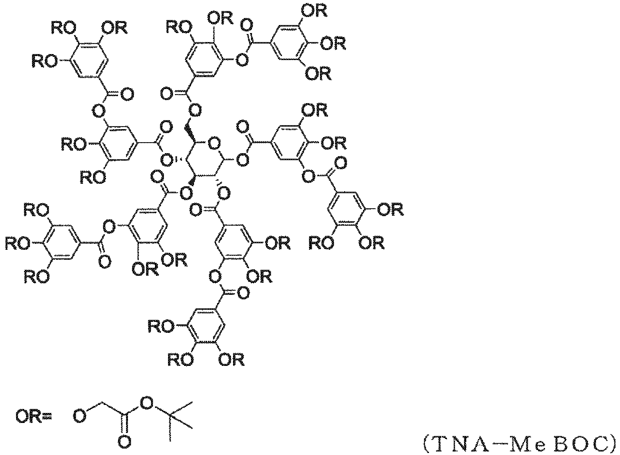

Also, the obtained compound (TNA-MeBOC) was evaluated for its solubility in a safe solvent by the above method. The results are shown in Table 1 (Example 5) .

### (Synthesis Example 6) Synthesis of ECT-BOC

The objective compound having a chemical structure of the following formula (ECT-BOC) was obtained in the same manner as in Synthesis Example 4 except that 3.34 g (11.5 mmol) of epicatechin having a structure of the following formula (ECT) (manufactured by Tokyo Kasei Kogyo Co., Ltd.) was used in place of tannic acid (TNA).

The following peaks were found by NMR measurement performed on the obtained compound under the above measurement conditions, and the compound was confirmed to have a chemical structure of the following formula (ECT-BOC).
δ (ppm) 2.7 (2H, -CH2-), 4.8-4.9 (2H, methine), 5.8-5.9 (2H, Ph-H), 6.6-6.7 (3H, Ph-H), 1.4 (9H, -CH3)

As a result of measuring the introduction rate of the acid dissociation reactive group in the obtained compound (ECT-BOC) by NMR, it was 100%.

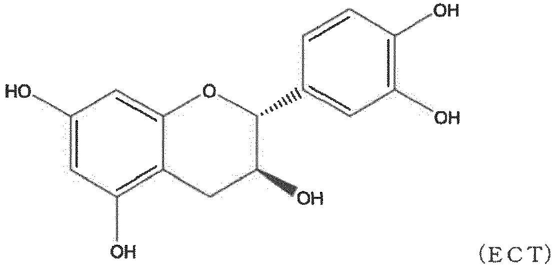

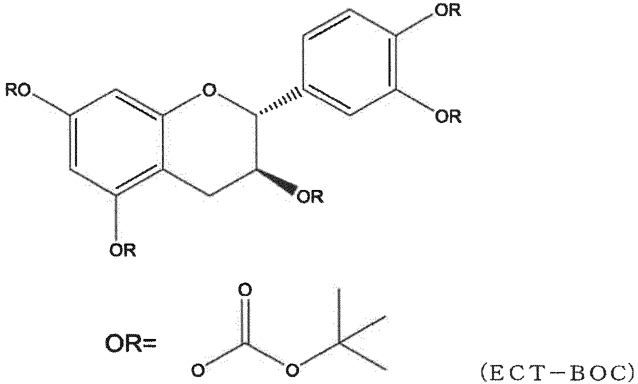

Also, the obtained compound (ECT-BOC) was evaluated for its solubility in a safe solvent by the above method. The results are shown in Table 1 (Example 6).

### (Comparative Synthesis Example 1) Synthesis of CR-1

A 4-neck flask (internal capacity: 10 L) equipped with a Dimroth condenser tube, a thermometer, and a stirring blade and having a detachable bottom was prepared. To this 4-neck flask, 1.09 kg (7 mol) of 1,5-dimethylnaphthalene (manufactured by Mitsubishi Gas Chemical Co., Inc.), 2.1 kg (28 mol as formaldehyde) of 40% by mass of an aqueous formalin solution (manufactured by Mitsubishi Gas Chemical Co., Inc.), and 0.97 mL of 98% by mass of sulfuric acid (manufactured by Kanto Chemical Co., Inc.) were fed in the current of nitrogen, and the mixture was reacted for 7 hours while being refluxed at 100°C at normal pressure. Subsequently, 1.8 kg of ethylbenzene (a special grade reagent manufactured by Wako Pure Chemical Industries, Ltd.) was added as a diluting solvent to the reaction solution, and the mixture was left to stand still, followed by removal of an aqueous phase as a lower phase. Neutralization and washing with water were further performed, and ethylbenzene and unreacted 1,5-dimethylnaphthalene were distilled off under reduced pressure to obtain 1.25 kg of a dimethylnaphthalene formaldehyde resin as a light brown solid.

Then, a 4-neck flask (internal capacity: 0.5 L) equipped with a Dimroth condenser tube, a thermometer, and a stirring blade was prepared. To this 4-neck flask, 100 g (0.51 mol) of the dimethylnaphthalene formaldehyde resin obtained as described above and 0.05 g of p-toluenesulfonic acid were fed in the current of nitrogen, the temperature was elevated to 190°C, and the mixture was heated for 2 hours and then stirred. Subsequently, 52.0 g (0.36 mol) of 1-naphthol was further added thereto, the temperature was further elevated to 220°C, and the mixture was reacted for 2 hours. After dilution with a solvent, neutralization and washing with water were performed, and the solvent was distilled off under reduced pressure to obtain 126.1 g of a modified resin (CR-1) as a black-brown solid.

### (Comparative Synthesis Example 2) Synthesis of CR-1-BOC

In a container (internal capacity: 200 mL) equipped with a stirrer, a condenser tube, and a burette, 10 g of the obtained compound (CR-1) and 5.5 g (25 mmol) of di-t-butyl dicarbonate (manufactured by Sigma-Aldrich) were fed to 100 mL of acetone, 3.45 g (25 mmol) of potassium carbonate (manufactured by Sigma-Aldrich) was added, and the contents were reacted by being stirred at 20°C for 6 hours to obtain a reaction solution. Next, the reaction solution was concentrated, and the reaction product was precipitated by the addition of 100 g of pure water to the concentrate, cooled to room temperature, and then filtered to separate solid matter.

The obtained solid matter was washed with water and dried under reduced pressure to obtain 4 g of a modified resin (CR-1-BOC) as a black solid.

Moreover, the solubility of the obtained compound (CR-1-BOC) in a safe solvent was evaluated by the above measurement method. The results are shown in Table 1 (Comparative Example 1).

### [Examples and Comparative Examples]

### (Preparation of Resist Composition)

A resist composition was prepared according to the formula shown in Table 1 below using each of the compounds synthesized in Synthesis Examples and Comparative Synthesis Examples. Among the components of the resist composition in Table 1, the following acid generating agent (P), acid diffusion controlling agent (E), and solvent (S-1) were used.

### [Acid Generating Agent (P)]

P-1: triphenylsulfonium trifluoromethanesulfonate (Midori Kagaku Co., Ltd.)

### [Acid Diffusion Controlling Agent (E)]

E-1: trioctylamine (Tokyo Kasei Kogyo Co., Ltd.)

### [Solvent]

S-1: propylene glycol monomethyl ether acetate (Tokyo Kasei Kogyo Co., Ltd.)

Each evaluation of the obtained resist compositions was carried out by the above measurement method. The results are shown in Table 1.

**[Table 1]**

| | | Safe solvent solubility test | Resist compositions | | | | | | | |
|---|---|---|---|---|---|---|---|---|---|---|
| | | | Composition | | | | Evaluation | | | |
| | | | Compound [g] | Acid generating agent (P) P-1 [g] | Acid diffusion controlling agent [g] | Solvent S-1 [g] | Storage stability | Thin film formability | Sensitivity evaluation | Pattern formation |
| Example 1 | TNA-ADBAC | A | 1 | 0.3 | 0.03 | 50 | A | A | A | A |
| Example 1-2 | TNA-ADBAC | A | 1 | 0.3 | 0 | 50 | A | A | A | A |
| Example 2 | TNA-ADBAC 100 | A | 1 | 0.3 | 0.03 | 50 | A | A | A | A |
| Example 3 | TNA-BVE | A | 1 | 0.3 | 0.03 | 50 | A | A | A | A |
| Example 4 | TNA-BOC | A | 1 | 0.3 | 0.03 | 50 | A | A | A | A |
| Example 5 | TNA-MeBOC | A | 1 | 0.3 | 0.03 | 50 | A | A | A | A |
| Example 6 | ECT-BOC | A | 1 | 0.3 | 0.03 | 50 | A | A | A | B |
| Comparative Example 1 | CR-1-BOC | A | 1 | 0.3 | 0.03 | 50 | A | A | C | C |

As can be understood from Table 1, the compounds used in Examples 1 to 6 (the compounds synthesized in Synthesis Examples 1 to 6) were able to be confirmed to have excellent solubility in a safe solvent at the same level as in the compound used in Comparative Example 1 (the compounds synthesized in Comparative Synthesis Example 2).

As a result of evaluating thin film formability according to the above measurement method, the resist compositions obtained in Examples 1 to 6 were able to form an excellent thin film at the same level as in Comparative Example 1.

Pattern evaluation (sensitivity and pattern shape evaluation) was carried out by the above measurement method using the resist compositions obtained in Examples and Comparative Example. In Example 1, a good positive type resist pattern was obtained by irradiation with electron beams of 1:1 line and space setting with a 50 nm interval. As can also be understood from comparison with Comparative Example 1, the resist compositions obtained in Examples 1 to 6 were excellent in both sensitivity and pattern shape.

From the above results, it was found that the compound according to the present embodiment has high solubility in safe solvents, and, also, a resist composition containing the compound has good storage stability, excellent thin film formability, and high sensitivity and can impart an excellent shape to a resist pattern, as compared with a resist composition containing the comparative compound (CR-1-BOC).

As long as the requirements of the present invention are met, compounds other than the compounds described in Examples also exhibit the same effects.

As mentioned above, the resist composition of the present invention and a pattern formation method using the same, and the compound and the resin of the present invention can be used widely and effectively in, for example, electrical insulating materials, resins for resists, encapsulation resins for semiconductors, adhesives for printed circuit boards, electrical laminates mounted in electric equipment, electronic equipment, industrial equipment, and the like, matrix resins of prepregs mounted in electric equipment, electronic equipment, industrial equipment, and the like, buildup laminate materials, resins for fiber-reinforced plastics, resins for encapsulation of liquid crystal display panels, coating materials, various coating agents, adhesives, coating agents for semiconductors, resins for resists for semiconductors, and resins for underlayer film formation.

The disclosure of Japanese Patent Application No. 2017-072550 filed on March 31, 2017 is incorporated herein by reference in its entirety.

All literatures, patent applications, and technical standards described herein are incorporated herein by referent to the same extent as if each individual literature, patent application, or technical standard is specifically and individually indicated to be incorporated by reference.

## Claims

1. A resist composition comprising one or more tannin compounds selected from the group consisting of a tannin comprising at least one acid dissociation reactive group in the structure and a derivative thereof, and a resin obtained using the tannin or the derivative as a monomer.

2. The resist composition according to claim 1, wherein the tannin compounds comprise one or more selected from the group consisting of a compound represented by the following formula (0), and a resin obtained using the compound represented by the following formula (0) as a monomer: 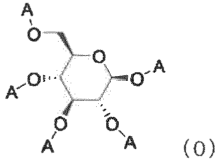 wherein each A is independently a hydrogen atom, or any structure represented by the following formula (A), provided that at least one A is any structure represented by the following formula (A): 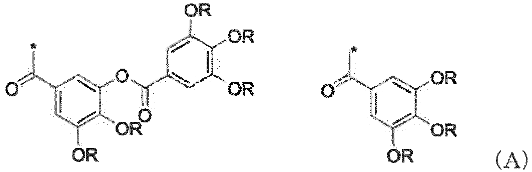 wherein each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group; and * indicates a point of attachment to the formula (0).

3. The resist composition according to claim 1, wherein the tannin compounds comprise one or more selected from the group consisting of a compound represented by the following formula (0-1) or the following formula (0-2), and a condensate having a structure derived from the compound represented by the following formula (0-1) and/or the following formula (0-2), and a resin obtained using the compound or the condensate as a monomer: 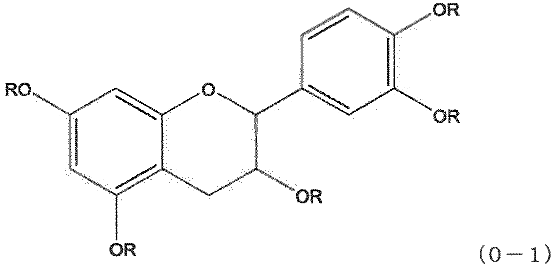 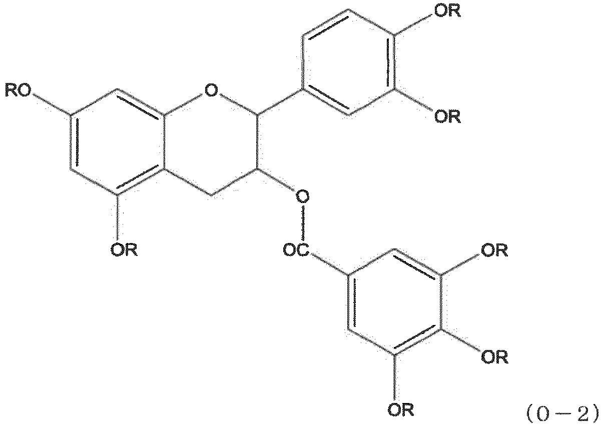 wherein each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group.

4. The resist composition according to claim 2, wherein the compound represented by the above formula (0) is a compound represented by the following formula (1): 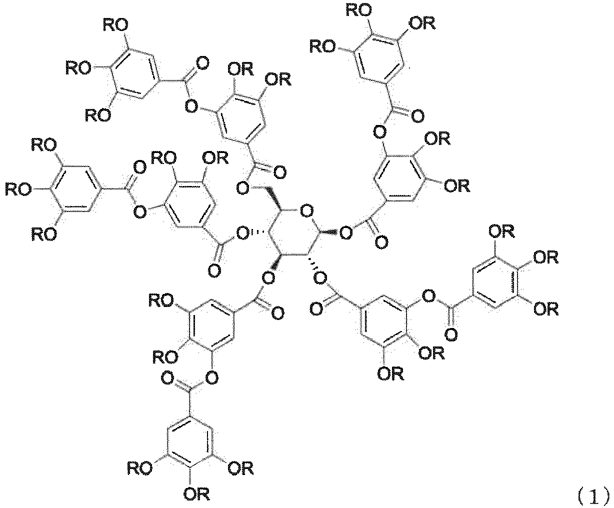 wherein each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provide that at least one R is an acid dissociation reactive group.

5. The resist composition according to any one of claims 1 to 4, further comprising a solvent.

6. The resist composition according to any one of claims 1 to 5, further comprising an acid generating agent.

7. The resist composition according to any one of claims 1 to 6, further comprising an acid diffusion controlling agent.

8. A method for forming a pattern, comprising the steps of:
forming a resist film on a substrate using the resist composition according to any one of claims 1 to 7;
exposing the resist film; and
developing the resist film, thereby forming a pattern.

9. A compound represented by the following formula (0): 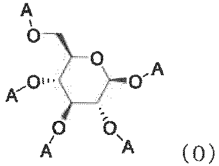 wherein each A is independently a hydrogen atom, or any structure represented by the following formula (A), provided that at least one A is any structure represented by the following formula (A): 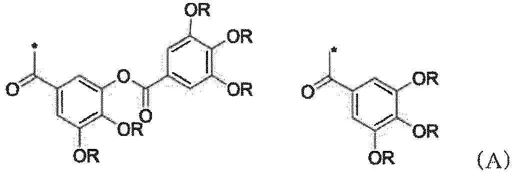 wherein each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group; and * indicates a point of attachment to the formula (0).

10. A resin obtained using the compound according to claim 9 as a monomer.

11. A compound selected from the group consisting of a compound represented by the following formula (0-1) or the following formula (0-2), and a condensate having a structure derived from the compound represented by the following general formula (0-1) and/or the following general formula (0-2): 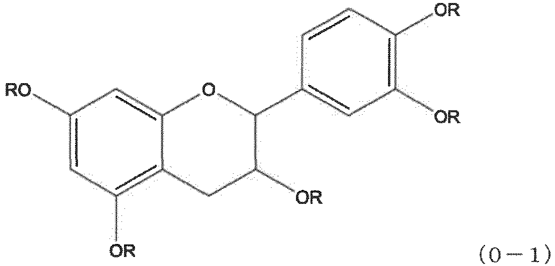 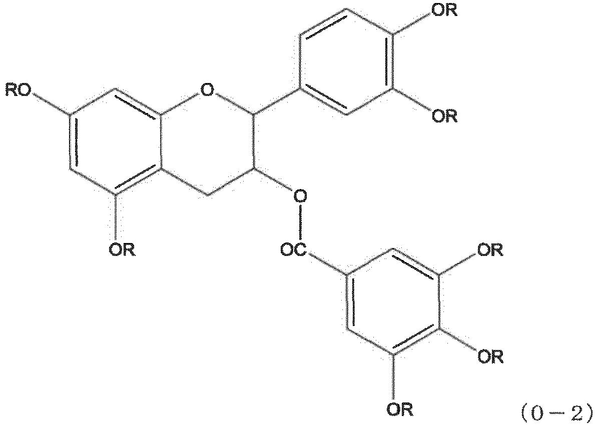 wherein each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group.

12. A resin obtained using the compound according to claim 11 as a monomer.

13. A compound represented by the following formula (1): 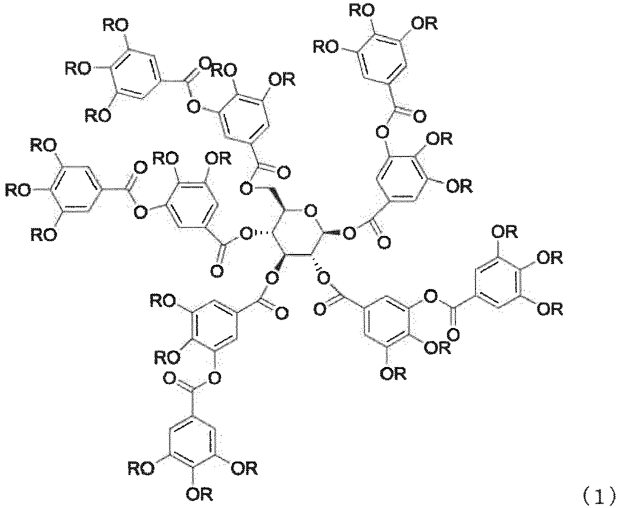 wherein each R is independently a hydrogen atom, a substituted or unsubstituted linear alkyl group having 1 to 20 carbon atoms, a substituted or unsubstituted branched alkyl group having 3 to 20 carbon atoms, a substituted or unsubstituted cyclic alkyl group having 3 to 20 carbon atoms, a substituted or unsubstituted aryl group having 6 to 20 carbon atoms, a substituted or unsubstituted alkenyl group having 2 to 20 carbon atoms, a halogen atom, or an acid dissociation reactive group, provided that at least one R is an acid dissociation reactive group.

14. A resin obtained using the compound according to claim 13 as a monomer.
